(19) Europäisches Patentamt
European Patent Office
Office européen des brevets

(11) **EP 2 679 991 B1**

(12) **EUROPÄISCHE PATENTSCHRIFT**

(45) Veröffentlichungstag und Bekanntmachung des Hinweises auf die Patenterteilung:
**15.12.2021 Patentblatt 2021/50**

(51) Int Cl.:
*C03B 5/235* $^{(2006.01)}$    *C03B 5/237* $^{(2006.01)}$
*G01N 33/00* $^{(2006.01)}$

(21) Anmeldenummer: **13169976.1**

(22) Anmeldetag: **31.05.2013**

(54) **Verfahren zur Bestimmung eines Kohlenmonoxid-Anteils in einem Abgasstrom, insbesondere eine Steuereinrichtung und einem regenerativ befeuerten Industrieofen**

Method for determining the carbon monoxide content in an exhaust gas stream, in particular a control unit and a regeneratively fired industrial furnace

Procédé de détermination d'un taux de monoxyde de carbone dans un flux de gaz d'échappement, notamment un dispositif de commande et un four industriel régénératif

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.06.2012 DE 102012210753**

(43) Veröffentlichungstag der Anmeldung:
**01.01.2014 Patentblatt 2014/01**

(73) Patentinhaber: **Software & Technologie Glas GmbH (STG)**
**03050 Cottbus (DE)**

(72) Erfinder:
• **Hemmann, Peter**
**03050 Cottbus (DE)**

• **Schulz, Thomas**
**03159 Döbern (DE)**
• **Heelemann, Helmut**
**03050 Cottbus (DE)**

(74) Vertreter: **Eisenführ Speiser**
**Patentanwälte Rechtsanwälte PartGmbB**
**Stralauer Platz 34**
**10243 Berlin (DE)**

(56) Entgegenhaltungen:
DE-A1- 4 323 879      DE-A1-102010 041 155
JP-A- 2012 026 718

**Beschreibung**

Die Erfindung betrifft ein Verfahren zur Bestimmung eines Kohlenmonoxid-Anteils in einem Abgasstrom eines regenerativ befeuerten Industrieofens nach dem Oberbegriff des Anspruchs 1, insbesondere eines Glasschmelzofens. Die Erfindung betrifft auch eine Steuereinrichtung und einen regenerativ befeuerten Industrieofen gemäß den Ansprüchen 17 und 18. Grundsätzlich ist ein Industrieofen nicht auf eine Verwendung in der Glasherstellung beschränkt. Zum Beispiel kann ein Industrieofen der eingangs genannten Art auch in der Metallherstellung oder dergleichen Herstellung eingesetzt werden. Ein regenerativer Industrieofen der eingangs genannten Art hat sich jedoch als besonders geeignet in der Glasherstellung zum Schmelzen von Glas erwiesen. Eine Steuerung, beispielsweise regenerativer Glasschmelzöfen, kann über einen Oberofenraum als Regelstrecke erfolgen. Bekannt ist beispielsweise ein Verfahren der Anmelderin aus DE 10 2010 041 155 A1 oder ein Verfahren aus JP 2012-26718. Eine Oberofentemperatur wird etwa durch eine Brennstoffmenge und/oder eine Verbrennungsluftmenge geregelt, beispielsweise durch Einstellen eines Verhältnisses zwischen Brennstoffmenge und Luftmenge. Dazu können Messwerte einer oder mehrerer Temperatur-Messsonden und einer oder mehrerer Lambda-Messsonden zur Verfügung gestellt werden. Eine Sauerstoffzugabe zum Oberofenraum und/oder Brennraum eines Industrieofens, z.B. für eine Glasschmelzwanne eines vorgenannten regenerativen Glasschmelzofens, erfolgt im Interesse möglichst hoher Energieeffizienz; damit zumeist mit einem geringen Luftüberschuss. Entsprechende Lambda-Werte einer Lambda-Sonde liegen z.B. zwischen 1,03 und 1,07. Regelmäßig wird in diesem Bereich ein stöchiometrischer Mindest-Sauerstoffbedarf für die gegebene Brennstoffmenge zum Betrieb eines Glasschmelzofens festgelegt. Der stöchiometrische Luftbedarf ergibt sich aus der Brennstoffanalyse. Der Betreiber eines Glasschmelzofens oder dergleichen Industrieofen kann einen kleinen Luftüberschuss darüber für seinen Betrieb festlegen, der ofenspezifisch ist, um gerade eine vollständige Verbrennung zu erreichen und möglichst einen Kohlenmonoxid-Anteil gering zu halten. Je geringer dabei ein Luftüberschuss ist, desto weniger Überschussluft muss durch das Feuerungssystem zusätzlich auf die Temperatur des Abgases aufgeheizt werden.

Andererseits zeigt sich, dass sich bei geringer werdendem Luftüberschuss eine zunehmend höher werdende Konzentration an Kohlenmonoxid einstellt. Kohlenmonoxid bildet die begrenzende Bedingung für die an sich energetisch vorteilhafte Verknappung des Luftüberschusses bei der Beheizung eines Industrieofens. Eine steigende Konzentration an Kohlenmonoxid verursacht ihrerseits energetische Verluste aufgrund der im Kohlenmonoxid gebundenen chemischen Energie. Weitaus kritischer als dieser Energieverlust ist jedoch die Gefährdung des Feuerfestmaterials des Industrieofens, insbesondere des feuerfesten Materials bei einer Glasschmelzwanne, durch Kohlenmonoxid-Anteile im Abgas, insbesondere bei über das chemische Gleichgewicht hinaus deutlich überhöhten Kohlenmonoxid-Konzentrationen. Eine stöchiometrische CO-Konzentration sollte immer genau Null sein, wird aber praktisch niemals erreicht. Insofern stellt jede CO-Konzentration eine Abweichung vom stöchiometrischen Zustand dar, verursacht durch das Schlupfloch eines chemischen Wasser-Gas-Gleichgewichts und schlimmerenfalls verursacht durch mangelhafte Verbrennung im Ofen. Es hat sich gezeigt, dass Kohlenmonoxid einen nachhaltig nachteiligen Einfluss auf die Struktur des feuerfesten Materials hat und zu einer erheblichen Lebenszeitverringerung des Industrieofens beitragen kann. Dies gilt insbesondere für drastisch erhöhte Kohlenmonoxidwerte im dreistelligem ppm-Bereich.

Die Bestimmung eines Kohlenmonoxid-Anteils in einem Abgasstrom eines regenerativ beheizten Industrieofens erweist sich somit als wichtige Maßnahme zum nachhaltigen Betrieb eines Industrieofens. Eine Möglichkeit, einen Kohlenmonoxid-Anteil theoretisch zu bestimmen besteht darin, einen Sauerstoff-Anteil, insbesondere als Sauerstoff-RestKonzentration zu bestimmen sowie dazu einen Kohlenmonoxid-Anteil, insbesondere als zugehörige Kohlenmonoxid-Konzentration. Beispielhaft ist dies in DE 43 23 879 A1 erläutert am Ergebnis einer Messung mit einer Zirkonoxid-Lambda-Sonde im Abgasstrom nach dem Feuerraum des Industrieofens.

Grundsätzlich ist dazu eine erste Berechnungsvorschrift zur Ermittlung eines Sauerstoff-Anteils im Abgas und eine zweite Berechnungsvorschrift zur Ermittlung eines Kohlenmonoxid-Anteils im Abgas bekannt, beispielsweise gemäß der Veröffentlichung von Möbius et al. in Sensor 95, Nürnberg, Deutschland, Kongressveröffentlichung, S. 845 - 848 (ACS 1995) --auch in 81th Communication on Oxide-Ion-Conducting Solid Electrolytes and their possible Application und 80th Communication in Solid State Phenomena 39 - 40 (1994, S. 229-272).

Eine Zirkonoxidsonde ist grundsätzlich beschrieben in: Zirox Sensoren&Elektronik GmbH Informationsblatt 1995 "Grundlagen der Anwendung potentiometrischer $ZrO_2$-Festelektrolytsensoren bei der optimalen Führung von Verbrennungsprozessen".

Grundlage der vorgenannten Berechnungsverfahren ist die Berechnung einer Sauerstoff-Konzentration mit Hilfe einer auf der Nernst'schen-Gleichung basierenden Berechnungsvorschrift --hier in der Form ln ($O_2$ %) = 3.0417 - 46.4188 * U/(T+273)-- dies unter Nutzung der Zellspannung U (mV) und Temperatur T(°C) einer Zirkonoxid-Sonde. Unter Zuhilfenahme einer zweiten Berechnungsvorschrift auf Basis einer Gleichung für ein Wasser-Gas-Gleichgewicht kann ein Verhältnis aus Kohlenmonoxid zu Kohlendioxid ($CO/CO_2$) für dieselbe Zellspannung bestimmt werden; also ein Kohlenmonoxid-Anteil. Dabei gehen Berechnungsvorschriften einer Verbrennungsrechnung ein, insbesondere bei Kenntnis der chemischen Zusammensetzung des Brennstoffes, sodass sich insgesamt ein Kohlenmonoxid-Anteil berechnen lässt. Die vorgenannten Berechnungsvorschriften --Nernst'sche Gleichung und/oder Wasser-Gas-Gleichung-- sind mit Hinweis auf obige DE 43 23 879 A1 insbesondere für das chemische Gleichgewicht eines Gasgemisches aussagekräftig. Weitere relevante Dokumente in diesem technischen Gebiet sind DE 36 32 480 A1, DE 10 2010 041 155 A1 und JP2012026718 A.

Bislang wurde angenommen, dass sich Gas-Anteile in einem Abgasstrom eines regenerativ befeuerten Industrieofens basierend auf dem chemischen Gleichgewicht beschreiben lassen. Zum Einen zeigt sich jedoch bei genauerer Analyse, dass die Abgasströme-insbesondere bei wechselnder Befeuerung bzw. bei wechselnder regenerativer Zuführung von Verbrennungsluft in einer ersten Periodendauer von einem linken Regenerator und in einer zweiten Periodendauer von einem rechten Regenerator-- durchaus Zeitskalen ergeben, die ein chemisches Gleichgewicht einer Abgasmenge nicht notwendigerweise gerechtfertigt erscheinen lassen. So wechselt eine Befeuerung zwar regelmäßig auf einer vergleichsweise langen Skala von einigen zehn Minuten jedoch dauert die Durchführung einer Gasströmung von einem linken Regenerator zu einem rechten Regenerator nur bis zu einer Minute. Gegebenenfalls ist dies nicht ausreichend dafür, dass sich ein chemisches Gleichgewicht einstellt.

Darüberhinaus kann für jeden Industrieofen die Annahme von optimalen Verbrennungsbedingungen gelten. Es zeigt sich --insbesondere für Situationen mangelhafter Verbrenneinstellungen oder unzweckmäßiger Führung der Brennstoff- und Verbrennungsluftströme im Ofenraum-- dass sich letztlich aufgrund mangelhafter Mischung von Brennstoff und Sauerstoff oder auf andere Weise eine verzögerte Verbrennungsdynamik trotz an sich ausreichender Konstruktion des Oxidationsmittels Sauerstoff einstellen kann. Dies kann zu einer deutlich erhöhten Konzentration von Kohlenmonoxid führen, d.h. deutlich über dem Gleichgewichts-Anteil von Kohlenmonoxid. Solche und andere praxisrelevante Situationen, in denen das Abgas nach dem Feuerraum das chemische Gleichgewicht eben nicht mehr erreicht oder ihm nicht ausreichend nahekommen kann führen dazu, dass sich die oben genannte Methodik zur Bestimmung eines Kohlenmonoxid-Anteils als unzureichend erweist. Insbesondere zeigt sich, dass bei einem Verfahren zur Steuerung und/oder Regelung eines regenerativ befeuerten Industrieofens unter Berücksichtigung eines Kohlenmonoxid-Anteils die Bestimmung des Kohlenmonoxid-Anteils noch verbesserungswürdig ist.

An dieser Stelle setzt die Erfindung an, deren Aufgabe es ist, ein Verfahren zur Bestimmung eines Kohlenmonoxid-Anteils in einem Abgasstrom sowie eine Vorrichtung anzugeben, mittels der eine verbesserte Bestimmung des Kohlenmonoxid-Anteils möglich ist. Insbesondere soll ein verbessertes Verfahren zur Steuerung und/oder Regelung eines regenerativ befeuerten Industrieofens angegeben werden. Insbesondere soll eine verbesserte Steuerung und ein verbesserter Industrieofen angegeben werden.

Die Aufgabe betreffend das Verfahren wird durch ein Verfahren des Anspruchs 1, insbesondere einem Verfahren zur Steuerung und/oder Regelung eines regenerativ befeuerten Industrieofens, gelöst. Das Konzept der Erfindung führt auch auf ein Verfahren zum Betrieb, insbesondere zum gesteuerten und/oder geregelten Betrieb eines Industrieofens, vorzugsweise eines regenerativ befeuerten Industrieofens unter Nutzung des Verfahrens des Anspruchs 1 zur Bestimmung eines Kohlenmonoxid-Anteils in einem Abgasstrom. Die Aufgabe betreffend die Vorrichtung wird durch eine Steuereinrichtung für einen regenerativ befeuerten Industrieofen des Anspruchs 17 gelöst, die ausgebildet ist zur Ausführung eines Verfahrens nach dem Konzept der Erfindung. Diese kann eine oder mehrere Hardware- und/oder Softwaremodule aufwiesen, die ausgebildet sind, einen oder mehrere der Verfahrensschritte gemäß dem Konzept auszuführen.

Die Aufgabe betreffend die Vorrichtung wird auch durch einen regenerativ befeuerten Industrieofen des Anspruchs 18 gelöst, insbesondere einen Glasschmelzofen mit einer Steuereinrichtung der vorgenannten Art.

Die Erfindung geht von der Überlegung aus, dass für eine schadstoffarme, energetisch sparsame und dennoch schonende Fahrweise eines Industrieofens --vorzugsweise neben einer möglichst exakten Ermittlung und Regelung der Luftüberschusszahl Lambda-- auch die rechtzeitige Erkennung vorgenannter deutlich über dem Gleichgewichts-Anteil von Kohlenmonoxid CO auftretende Kohlenmonoxid-Anteile erforderlich ist. Vorzugsweise wird der zugehörige Sauerstoffanteil oder der zugehörige Luftüberschusswert (Lambda-Wert) im Gleichgewicht mit Hilfe des Gleichgewichtswertes ermittelt. Allerdings lässt sich, wie von der Erfindung erkannt, ein zweiter, einen Nichtgleichgewichts-Anteil kennzeichnender, Kohlenmonoxid-Anteil im Abgas auch ohne Bestimmung eines Gleichgewichts-Anteils von Sauerstoff bestimmen.

Auch geht die Erfindung von der Überlegung aus, dass eine Messung und Regelung des aktuellen Lambda-Wertes nicht notwendigerweise mit gutem Erfolg durch eine Messung und darauf aufbauende Regelung der Kohlenmonoxid-Konzentration ersetzt werden kann, weil die Messung des Kohlenmonoxids keinen direkten Rückschluss auf die tatsächlich vorhandene Verbrennungsluft zulässt. Insbesondere trägt dazu die Überlegung bei, dass beispielsweise ein PID-Such-Algorithmus --gemessen an der realen Verbrennungsdynamik-- zu langsam wäre, um passende Luftmengen zu finden. Insbesondere kann die Messung des Kohlenmonoxids keinen direkten Rückschluss zulassen, weil nur die Kohlenmonoxid-Indikation nicht an Stelle sondern nur zusätzlich zur Lambda-Messung eine getrennte Bewertung des Einflusses mangelhafter Verbrennungsdynamik ermöglicht.

Die Erfindung basiert somit auf der Überlegung, dass es erforderlich ist, die Ursache einer deutlich über dem Gleichgewichts-Kohlenmonoxid-Anteil liegenden Ungleichgewichts-Kohlenmonoxid-Anteil zu berücksichtigen, insbesondere für eine Steuerung und/oder Regelung eines Industrieofens. Es wurde erkannt, dass in diesem Fall Luftmangel einerseits und mangelhafte Verbrennungsdynamik trotz ausreichenden Luftangebots andererseits unterschiedliche Reaktionen verlangen und daher vorzugsweise einer regelungstechnisch separaten Berücksichtigung bedürfen.

Die regelungstechnische Kontrolle über die stabile Einhaltung eines niedrigen Luftüberschusses erfordert vorteilhaft eine in situ -Messung --d.h. als Funktion der Zeit-- der aktuellen Sauerstoff-Konzentration im Abgasstrom und die daraus hervor gehende Werteermittlung des aktuellen Luftüberschusses Lambda. Berechtigte Bedenken hinsichtlich einer überhöhten Konzentration an Kohlenmonoxid, verlangen --vorzugsweise zusätzlich-- eine Messung der Kohlenmonoxid-Konzentration am gleichen Messort. Somit geht eine Weiterbildung davon aus, dass mittels einer ersten Berechnungsvorschrift für einen Sauerstoff-Anteil im Abgas der Sauerstoff-Anteil ermittelt wird.

Gemäß dem Konzept der Erfindung wird mittels einer zweiten Berechnungsvorschrift für einen Kohlenmonoxid-Anteil im Abgas der Kohlenmonoxid-Anteil im Abgas ermittelt. Die Erfindung hat darüberhinaus erkannt, dass ein Nichtgleichgewichts-Anteil von Kohlenmonoxid unabhängig von einem Gleichgewichts-Anteil von Kohlenmonoxid bestimmt werden sollte. Das Konzept sieht --vereinfacht gesagt-- bevorzugt zwei separate Auswertungen vor; eine zur Ermittlung eines ersten, einen Gleichgewichts-Anteil, insbesondere eine Volumen-Konzentration, kennzeichnenden Kohlenmonoxid-Anteils im Abgas und eine weitere zur Ermittlung eines zweiten, einen Nichtgleichgewichts-Anteil kennzeichnenden, Kohlenmonoxid-Anteils im Abgas. Dabei wurde erkannt, dass zwei separate reale Messungen am gleichen Messort bei Ofentemperaturen von über 1400°C und unter chemisch aggressiven Umgebungsbedingungen nur mit vergleichsweise hohem technischen Aufwand zu realisieren wären. Einer verbesserten theoretischen Ermittlung des Kohlenmonoxids basierend auf einer Lambda-Messung ist damit der Vorzug zu geben.

Eine separate Berechnung eines Gleichgewichts-Anteils und eines Nichtgleichgewichts-Anteils von Kohlenmonoxid ist nach der Erkenntnis der Erfindung aus einem Datensatz, nämlich aus der Zellspannung möglich, insbesondere einer Anzahl von Zellspannungswerten als Funktion der Zeit.

Überraschend wurde erkannt, dass sich jedenfalls aufgrund mangelnder Verbrennungsdynamik überhöhte Kohlenmonoxid-Konzentrationen in den Zellspannungen als zeitlich beschränkte und wiederkehrende Zellspannungserhöhungen, insbesondere als Zellspannungsspitzen, erkennbar machen. Die Erfindung hat erkannt, dass diese zusätzlich auftreten zur überwiegend gemessenen Sauerstoff-Konzentration im Abgasstrom, wobei jedoch beide Signal-Anteile separat auswertbar sind. Der Messdatenstrom an Zellspannungswerten --zur vorzugsweisen Bestimmung einer Sauerstoff-Konzentration-- wird durch bisher unberücksichtigt gebliebene Zellspannungserhöhungen, insbesondere vergleichsweise sehr kurze Zellspannungsspitzen, überlagert, die erfindungsgemäß der über das chemische Gleichgewicht hinaus deutlich überhöhten Kohlenmonoxid-Konzentration zugeordnet werden.

Unabhängig von der konkreten Form der Signal-Anteile hat die Erfindung erkannt, dass sich in einem oben beschriebenen Fall eines Nichtgleichgewichts-Anteils von Kohlenmonoxid aus der Zellspannung ein Gleichgewichtswert ermitteln lässt und ein gegenüber dem Gleichgewichtswert deutlich erhöhter Nicht-Gleichgewichtswert ermitteln lässt. In dem genannten Fall wird davon ausgegangen, dass das Abgas kein homogenes Gasgemisch darstellt sondern die Lambda-Sonde --bildlich ausgedrückt-- z.B. von einer Folge von Turbulenz-Clustern passiert werden kann (diese mit deutlich unterschiedlicher chemischer Zusammensetzung). Die Erfindung geht davon aus, dass sich bei der Signalauswertung die erhöhten Nicht-Gleichgewichtswerte weitgehend unabhängig von den Gleichgewichtswerten auswerten lassen für den gleichen Abgasstrom. Tatsächlich ist damit die Möglichkeit gegeben, sowohl das dominierende Basisniveau der Zellspannung wie üblich auszuwerten, um die vorhandene Sauerstoff-Konzentration einschließlich des Gleichgewichts-Kohlenmonoxids zu bestimmen; als auch die dazu nicht im Gleichgewicht befindliche überhöhte Kohlenmonoxid-Konzentration aus beispielsweise mangelnder Verbrennungsdynamik oder anderen Ursachen. Dazu werden Beiträge zum Gleichgewichtswert und Beiträge zum erhöhten Nicht-Gleichgewichtswert separat herangezogen.

Besonders bevorzugte Weiterbildungen der Erfindung sind den Unteransprüchen zu entnehmen und geben im Einzelnen vorteilhafte Möglichkeiten an, das Konzept der Erfindung im Rahmen der Aufgabenstellung mit weiteren Vorteilen versehen weiterzubilden.

Insbesondere ist vorgesehen, dass aus den aufeinanderfolgenden Messdaten einer Zellspannung einer im zu analysierenden Abgasstrom befindlichen an sich bekannten Zirkonoxid-Festelektrolyt-Sonde zyklisch wiederkehrende Zellspannungsspitzen, die sich vom durchschnittlichen Basisniveau der Zellspannungswerte deutlich unterscheiden, abgetrennt und einer separaten Signalauswertung unterworfen werden. Bevorzugt stellt eine Wiederholfrequenz und Spannungshöhe der Zellspannungsspitzen ein Mass für die gegenüber dem chemischen Gleichgewicht überhöhte Volumenkonzentration an Kohlenmonoxid dar.

So ist es beispielsweise möglich, die Häufigkeit und Höhe der Zellspannungsspitzen gemäß dem vorgenannten Beispiel auszuwerten. Dazu können die beispielhaft genannten Zellspannungsspitzen aus dem Messdatenstrom separiert und erfasst werden; insbesondere ohne Vermischung mit einem Basisniveau von Zellspannungswerten. Es lässt sich eine für beide Signal-Anteile geeignete, im vorliegenden Fall insbesondere gleiche, Signalauswertung auf Basis der Nernst'schen Gleichung und der Wasser-Gas-Gleichung --beide für eine quasistatische Gleichgewichtssituation-- durchführen. Im Ergebnis kann neben dem Sauerstoff-Anteil und dem Gleichgewichts-Kohlenmonoxid-Anteil auch ein Nichtgleichgewichts-Kohlenmonoxid-Anteil angegeben werden.

Erfindungsgemäß wird zusätzlich zur Zellspannung der Lambda-Sonde eine Temperatur der Lambda-Sonde zur Verfügung gestellt und/oder eine Brennstoffzusammensetzung zur Verfügung gestellt. Insbesondere kann eine Abgas-Temperatur, insbesondere zusätzlich zur Temperatur der Lambdasonde, und eine Brennstoff-Zusammensetzung zur Verfügung gestellt werden. Der Brennstoff dient der Verbrennung mit Verbrennungsluft zur Beheizung des Industrieofens, insbesondere des Glasschmelzofens.

Im Rahmen der Erfindung ist die erste Berechnungsvorschrift eine Sondenvorschrift, d. h. eine gemäß einem Betriebsmodell der Lambda-Sonde ausgebildete Berechnungsvorschrift. Im Rahmen der Erfindung ist die Berechnungsvorschrift eine Funktion der Zellspannung und der Temperatur der Lambda-Sonde basierend auf einer Nernst'schen Gleichung. Vorzugsweise hat die auf einer Nernst'schen Gleichung basierende Berechnungsvorschrift die Form $\ln (O_2\text{-Ant}) = C1 - C2 \cdot U/(T+T0)$. Die dort gegebenen Konstanten C1 und C2 sind bevorzugt Konstanten, welche die Art der Lambda-Sonde, insbesondere nämlich eine Zirkonoxid-Festelektrolyt-Sonde, berücksichtigen. Insbesondere können die Konstanten auch einen Normal-Sauerstoff-Anteil der Luft berücksichtigen. Die Konstante T0 berücksichtigt die Temperaturdifferenz zum absoluten Nullpunkt und beträgt überlicherweise 273, wenn die Temperatur in Grad Celsius angegeben ist. Die Nernst'sche Gleichung hat sich als besonders bevorzugt erwiesen, um als Basis zur Beschreibung einer Zirkonoxid-Festelektrolyt-Sonde oder ähnlicher Lambda-Sonde zu dienen.

Erfindungsgemäß ist die zweite Berechnungsvorschrift für einen Kohlenmonoxid-Anteil im Abgas eine Verbrennungsvorschrift, welche die Brennstoff-Zusammensetzung nutzt auf Basis einer Wasser-Gas-Gleichung, vorzugsweise der Form $\ln (CO\_Ant/CO_2\_Ant) = D1 - D2 \cdot (U0-U)/(T+T0)$.

Die zweite Berechnungsvorschrift für einen Kohlenmonoxid-Anteil im Abgas ist eine Verbrennungsvorschrift, welche auch die Brennstoffzusammensetzung nutzt. Insbesondere ist die zweite Berechnungsvorschrift in der Form einer Verbrennungsvorschrift zusätzlich eine Funktion der Zellspannung und der Abgas-Temperatur und/oder der Temperatur der Lambda-Sonde; insbesondere der Abgas-Temperatur statt der Temperatur der Lambda-Sonde. Bevorzugt ist die zweite Berechnungsvorschrift in der Art einer Wasser-Gas-Gleichung, insbesondere für eine Gleichgewicht oder eine Quasi-Gleichgewichtssituation gebildet, vorzugsweise in der Form $\ln (CO\text{-Ant}/CO_2\text{-Ant}) = D1 - D2 \cdot (U0-U)/(T+T0)$. D1 und D2 berücksichtigen dabei als Konstanten die Art der Lambda-Sonde. U0 berücksichtigt die durch weitere Gas-Anteile hervorgerufene Zellspannung. T0 ist die zuvor genannte Differenz zum absoluten Temperaturnullpunkt; bei Angabe von T in Grad Celsius nämlich 273. Bezüglich der vorgenannten Weiterbildungen wird insbesondere auf die Eingangs genannte Veröffentlichung von Möbius hingewiesen, wie auch in DE 43 23 879 A1 beschrieben. Unter anderem können demnach ein Redox-Quotient Q der Anteile von $H_2O + CO_2$ im Verhältnis zu $H_2 + CO$ sowie ein Kohlenstoff-Wasserstoff Verhältnis V, nämlich von $CO+CO_2$ im Verhältnis zu $H_2+H_2O$ als auch die Summe S der Konzentrationen der Gas-Wasser-Komponenten nämlich CO, $CO_2$, $H_2$ und $H_2O$ eingehen.

Vorzugsweise kann mittels der zweiten Berechnungsvorschrift zur Ermittlung eines zweiten, einen Nichtgleichgewichts-Anteil kennzeichnenden, Kohlenmonoxid-Anteils, ein Sauerstoff-Anteil im Abgas vernachlässigt werden oder unter Nutzung eines geringen bis vernachlässigbaren Sauerstoff-Anteils der Nichtgleichgewichts-Anteil von Kohlenmonoxid ermittelt werden. Die Weiterbildung geht vorteilhaft von der Annahme aus, dass sich Sauerstoff-Anteil nur gering fällt bzw. sich nur auswirkt in dem beschriebenen Szenario. Insbesondere kann zusätzlich zur Zellspannung eine Abgas-Temperatur anstatt der Temperatur der Lambda-Sonde verwendet werden. Auch dies hat sich als vorteilhaft erwiesen bei der Anwendung der zweiten Berechnungsvorschrift.

Im Rahmen einer besonders bevorzugten Weiterbildung erweist es sich auch als vorteilhaft, dass --zusätzlich zur Ermittlung eines zweiten, einen Nichtgleichgewichts-Anteil kennzeichnenden, Kohlenmonoxid-Anteils im Abgas-- zur Ermittlung eines ersten, einen Gleichgewichts-Anteil kennzeichnenden, Kohlenmonoxid-Anteils im Abgas

- mittels einer ersten Berechnungsvorschrift ein Sauerstoff-Anteil im Abgas ermittelt wird, und unter Nutzung eines nicht vernachlässigbaren Sauerstoff-Anteils im Abgas
- unter Nutzung des genannten Gleichgewichtswert, mittels der zweiten Berechnungsvorschrift ein Kohlenmonoxid-Anteil im Abgas ermittelt wird, welcher einen Gleichgewichts-Anteil, insbesondere eine Volumen-Konzentration, kennzeichnenden Kohlenmonoxid-Anteil im Abgas kennzeichnet.

Im Rahmen einer besonders bevorzugten Weiterbildung des Verfahrens wird für die Zellspannung als Funktion der Zeit ein bestimmtes Integralzeitfenster und/oder ein bestimmtes Differentialzeitfenster vorgegeben, wobei das Differentialzeitfenster kleiner ist als das Integralzeitfenster; d.h. insbesondere umfasst das Differentialzeitfenster weniger Zellspannungswerte als das Integralzeitfenster. Mit Hilfe der vorgenannten Zeitfenster lässt sich auf besonders bevorzugte Weise ein Gleichgewichtswert zur Ermittlung des Gleichgewichts-Anteils des Kohlenmonoxid-Anteils bzw. ein erhöhter Nicht-Gleichgewichtswert zur Ermittlung eines Nicht-Gleichgewichts-Anteils des Kohlenmonoxid-Anteils bestimmen. Bevorzugt lässt sich der Gleichgewichtswert auf der Zeitskala des bestimmten Integralzeitfensters bestimmen. Bevorzugt lässt sich der erhöhte Nicht-Gleichgewichtswert auf der Zeitskala eines Differentialzeitfensters bestimmen. Das Integralzeitfenster liegt bevorzugt in einem Bereich oberhalb von 100 Sekunden, insbesondere oberhalb von 200 Sekunden. Das Differentialzeitfenster liegt bevorzugt im Bereich unterhalb von 50 Sekunden, vorzugsweise unterhalb von 10 Sekunden besonders bevorzugt unterhalb von 5 Sekunden. Die Zeitfenster werden bevorzugt zur laufenden

**EP 2 679 991 B1**

Mittelwertbildung über die akuten Werte derselben genutzt.

Bevorzugt lässt sich der Gleichgewichtswert als Mittelwert aller Zellspannungswerte im Integralzeitfenster ermitteln. Insbesondere ist der Gleichgewichtswert als Funktion der Zeit ermittelbar, insbesondere als laufender Langzeitmittelwert, bevorzugt von Zellspannungswerten unterhalb eines Schwellbereichs. Beispielsweise lässt sich ein Integralzeitfenster als laufendes Zeitfenster über der Zellspannung als Funktion der Zeit definieren. Das Integralzeitfenster, bevorzugt der vorgenannten Breite, wird somit von Zellspannungswerten durchlaufen und die jeweils aktuellen Zellspannungswerte werden über das Integralzeitfenster ermittelt, so dass sich ein laufender Langzeitmittelwert von Zellspannungswerten ergibt.

Es hat sich gezeigt, dass für den Fall dass eine Fehleinstellung des Industrieofens vorliegt, in der Tat die den Nicht-Gleichgewichts-Anteil des Kohlenmonoxid-Anteils charakterisierenden Zellspannungsspitzen deutlich in der Unterzahl sind und somit den Langzeitmittelwert von Zellspannungswerten kaum beeinflussen. In dem Fall liegt in der Tat der Gleichgewichtswert deutlich unterhalb eines Schwellbereichs bzw. ein laufender Langzeitmittelwert von erhöhten Zellspannungswerten liegt deutlich oberhalb eines Schwellbereichs. Dies ist ein grundsätzlich anderes Szenario in Vergleich zu einer Normaleinstellung des Betriebsofens mit nicht erhöhten Kohlenmonoxid-Werten. Dies ist auch ein grundsätzlich anderes Szenario im Vergleich zu einer Sauerstoff-Mangelsituation, mit im Gleichgewicht erhöhtem Kohlenmonoxid-Anteil.

Bevorzugt wird der erhöhte Nicht-Gleichgewichtswert zur Ermittlung eines zweiten, einen Nichtgleichgewichtsanteil kennzeichnenden, Kohlenmonoxid-Anteils im Abgas nur dann genutzt, wenn der erhöhte Nicht-Gleichgewichtswert einen Schwellbereich übersteigt. Diese Weiterbildung trägt der Tatsache Rechnung, dass sich außerhalb einer Gleichgewichtseinstellung zyklisch wiederkehrende Zellspannungsspitzen ergeben, die sich vom durchschnittlichen Grundniveau der Zellspannungswerte deutlich unterscheiden.

Insbesondere kann auch der erhöhte Nicht-Gleichgewichtswert zur Ermittlung eines zweiten, einen Nichtgleichgewichtsanteil kennzeichnenden, Kohlenmonoxid-Anteils, im Abgas nur dann genutzt werden, wenn eine Abgastemperatur wenigstens 10K, vorzugsweise wenigstens 25K oberhalb einer Normaltemperatur liegt. Die Weiterbildung hat erkannt, das derartige Überkonzentrationen an Kohlenmonoxid über dem Gleichgewichtszustand typischerweise auch mit überhöhten Abgastemperaturen einhergehen. Es werden Temperaturerhöhungen bis zu 50K gegenüber "normalen" Ausbrandverhältnissen beobachtet. Kohlenmonoxid selbst ebenso wie die Übertemperaturen verursachen vermeidbare Energieverluste und drohen das Feuerfestmaterial des Ofens zu beschädigen. Im Rahmen einer zusätzlichen oder alternativen Weiterbildung der Erfindung wird ein erhöhter Nicht-Gleichgewichtswert als Mittelwert oder Enveloppenwert aller Zellspannungswerte oberhalb eines Schwellbereichs und in Differentialzeitfenster ermittelt. Insbesondere erfolgt eine Signalauswertung der Lambda-Sonde mit separierten Zellspannungsspitzen zur Bildung des erhöhten Zellspannungswertes.

Insbesondere hat es sich als vorteilhaft erwiesen, dass ein gegenüber dem Gleichgewichtswert erhöhter Nicht-Gleichgewichtswert als Funktion der Zeit ermittelt wird, insbesondere als laufender Langzeit Mittelwert von erhöhten Zellspannungswerten oberhalb eines Schwellbereichs. Im Rahmen der Erfindung werden zur Identifizierung der Zellspannungsspitzen eine einstellbare große Zahl von Zellspannungswerten --nämlich für ein Integralzeitfenster-- in einer elektronischen Auswertvorrichtung gleitend gespeichert. Es wird dann das Maximum oder ein Enveloppenwert der gespeicherten Zellspannungswerte zur Bestimmung eines aktuellen Kohlenmonoxid-Anteils genutzt.

Im Unterschied zu einem Maximalwert, der jeweils einen konkreten Zellspannungswert annimmt, handelt es sich bei einem Enveloppenwert um einen, der sich aus Mittelung der Maximalwerte ergibt. Der Enveloppenwert nimmt also nicht jedes Maximum der Zellspannungswerte an, sondern hat als Funktion der Zeit einen kontinuierlichen und langsamer variierenden Verlauf als die Maximalwerte bzw. die Zellspannungswerte.

Grundsätzlich ist es vorteilhaft, dass zur Bestimmung des erhöhten Nicht-Gleichgewichtswertes heranzuziehende Zellspannungswerte und/oder der erhöhte Nicht-Gleichgewichtswert um einen Faktor von gleich oder mehr als 1,5, vorzugsweise mehr als 2,0, vorzugsweise mehr als 2,5, vorzugsweise mehr als 3, insbesondere mehr als 4 übersteigt. Es hat sich gezeigt, dass diese in vergleichsweise kurzen Differentialzeitfenstern beschränkte Zellspannungsspitzen vorzugsweise eine Halbwertsbreite unterhalb von 5 Sekunden, insbesondere unterhalb von 2 Sekunden, insbesondere unterhalb von 1,5 Sekunden haben. Beispielsweise für den Fall einer verzögerten Verbrennung von Kohlenmonoxid-Konzentration liegen die Zellspannungsspitzen nicht nur geringfügig über der Gleichgewichts-Konzentration, d.h. einem Gleichgewichtswert einer Zellspannung. Vielmehr nimmt die Kohlenmonoxid-Konzentration in einem solchen Fall, das 20- bis 50-fache oder noch weit höhere Vielfache einer Basis-Konzentration von Kohlenmonoxid an. In Zellspannungswerten ausgedrückt bedeutet dies, dass Zellspannungswerte eines Faktors von gleich oder mehr als 4,0 des Gleichgewichtswertes erreicht werden können. Vereinfacht ausgedrückt sind die Zellspannungsspitzen vergleichsweise Amplitudenstark und schmal.

Weiter bevorzugt treten zur Bestimmung des erhöhten Nicht-Gleichgewichtswertes heranzuziehende Zellspannungswerte mit einer durchschnittlichen Rate von einem bis fünf Werten pro Minute auf. Insbesondere ist ein Integralzeitfenster derart gebildet, dass wenigstens im Mittel zwei Zellspannungswerte zur Bestimmung des erhöhten Nicht-Gleichgewichtswerts heranzuziehen sind.

6

Insbesondere ist es vorteilhaft, dass ein Schwellbereich einen Bereich von Zellspannungswerten erfasst, die zwischen einem 1,0-fachen des Gleichgewichtswertes und einem 1,5-fachen des Gleichgewichtswertes liegen.

Bevorzugt werden der erste Kohlenmonoxid-Anteil im Abgas aus dem Gleichgewichtswert und der zweite Kohlenmonoxid-Anteil im Abgas aus dem erhöhten Nicht-Gleichgewichtswert mittels der gleichen, wenigstens einen Berechnungsvorschrift ermittelt. Insbesondere ist vorgesehen, dass eine Anwendung einer Nernst'schen Gleichung und eine Anwendung einer Wasser-Gas-Gleichung für eine Bestimmung eines ersten Kohlenmonoxid-Anteils im Abgas aus dem Gleichgewichtswert auf dieselbe Weise erfolgt, wie für eine Bestimmung eines zweiten Kohlenmonoxid-Anteils.

Weiter hat es sich als bevorzugt erwiesen, dass zur Kalibrierung einer Kohlenmonoxidmessung im Vergleich mit einer anderen unabhängigen Kohlenmonoxid-Konzentrationsmessung am gleichen Messort die geeignete Einstellung der einstellbaren Anzahl von Zellspannungswerten/für ein Integralzeitfester und/oder die geeignete Annahme einer Temperaturdifferenz zwischen analysiertem Abgas und der Temperatur der Messsonde selbst verwendet wird.

Bei der Breite des Integralzeitfensters handelt es sich insbesondere um einen Parameter, der vorteilhaft genutzt werden kann, um möglichst gut separierte Gleichgewichtswerte und Nicht-Gleichgewichtswerte als Funktion der Zeit ermitteln zu können. Ebenso kann eine Temperaturdifferenz zwischen analysiertem Abgas und der Temperatur der Messsonde als Parameter genutzt werden, um möglichst gut separierte Gleichgewichtswerte und Nicht-Gleichgewichtswerte als Funktion der Zeit ermitteln zu können.

Ausführungsbeispiele der Erfindung werden nun nachfolgend anhand der Zeichnung beschrieben. Diese soll die Ausführungsbeispiele nicht nur notwendigerweise maßstäblich darstellen, vielmehr ist die Zeichnung, wo zur Erläuterung dienlich, in schematisierter und/oder leicht verzerrter Form ausgeführt. Im Hinblick auf Ergänzungen der aus der Zeichnung unmittelbar erkennbaren Lehren wird auf dem einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in der Zeichnung sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, der Zeichnung und/oder den Ansprüchen offenbarten Merkmale. Die allgemeine Idee der Erfindung ist nicht beschränkt auf die exakte Form oder das Detail der im folgenden gezeigten und beschriebenen bevorzugten Ausführungsform oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Nicht-Gleichgewichtswerte offenbart und beliebig einsetzbar und beanspruchbar sein.

Weitere Vorteile, Merkmale und Einzelheiten der Erfindung ergeben sich aus der nachfolgenden Beschreibung der bevorzugten Ausführungsbeispiele sowie anhand der Zeichnung; diese zeigt in:

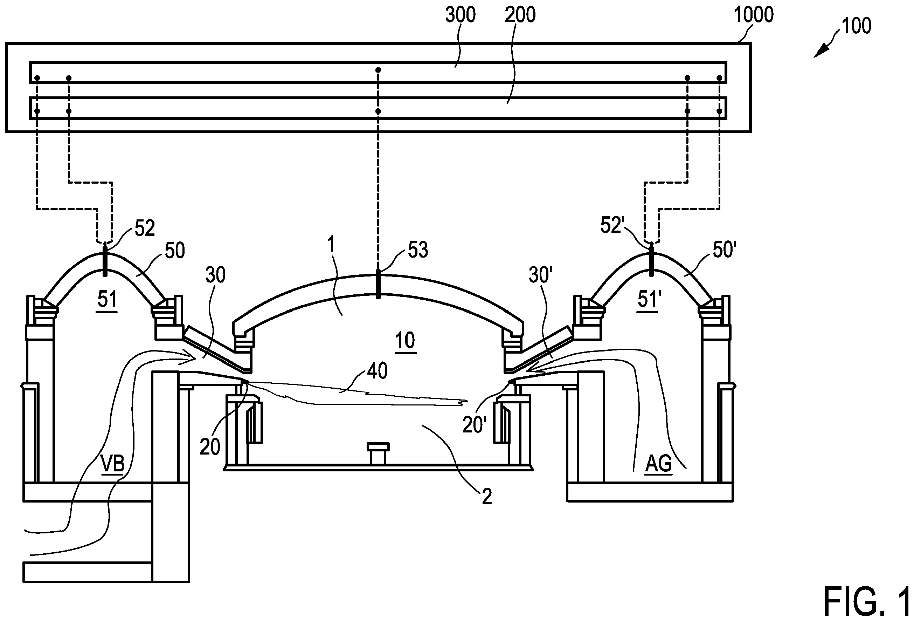

Fig. 1    eine schematische Darstellung eines regenerativ beheizten Industrieofens mit einem linken und einem rechten Regenerator gemäß einer besonders bevorzugten Ausführungsform, bei der eine Steuereinrichtung mit einem Temperaturregelmodul und einem Lambda-Regelmodul gemäß dem Konzept der Erfindung vorgesehen ist;

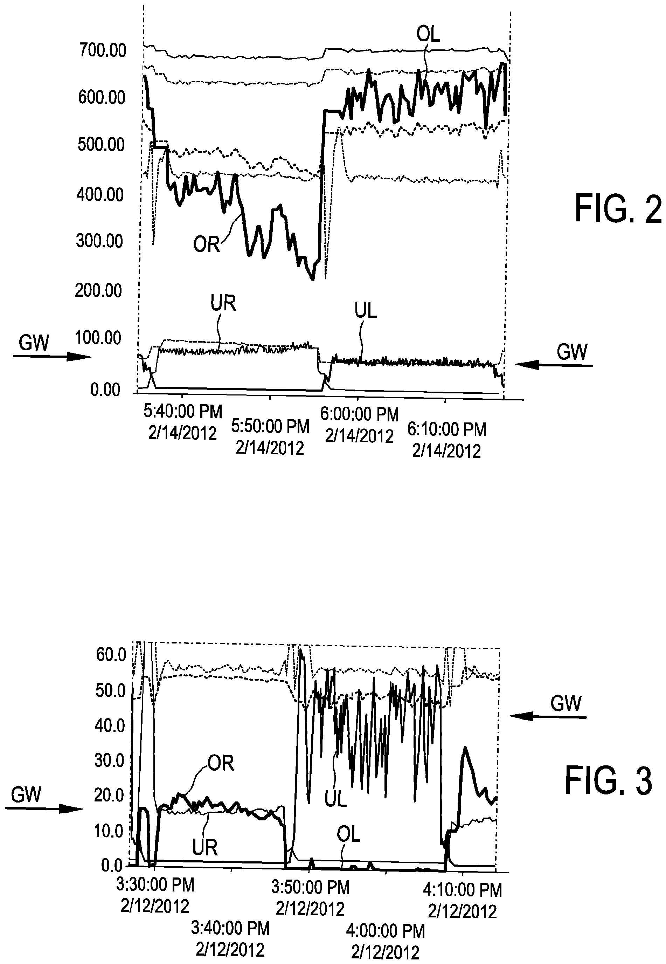

Fig. 2    eine beispielhafte Abfolge von Zellspannungswerten der in Fig. 1 gezeigten Lambda-Sonden zusammen mit dem über die Nernst'sche Gleichung und Wasser-Gas-Gleichung zur Lambda-Sonde bestimmten Sauerstoff-Anteil bei normaler Verbrennungssituation - d.h. im Gleichgewicht und ausreichendem Sauerstoffangebot;

Fig. 3    eine beispielhafte Abfolge von Zellspannungswerten der in Fig. 1 gezeigten Lambda-Sonden zusammen mit dem über die Nernst'sche Gleichung und Wasser-Gas-Gleichung zur Lambda-Sonde bestimmten Sauerstoff-Anteil bei einer Verbrennungssituation unter Sauerstoffmangel - d.h. im Gleichgewicht und mit nicht ausreichendem Sauerstoffangebot;

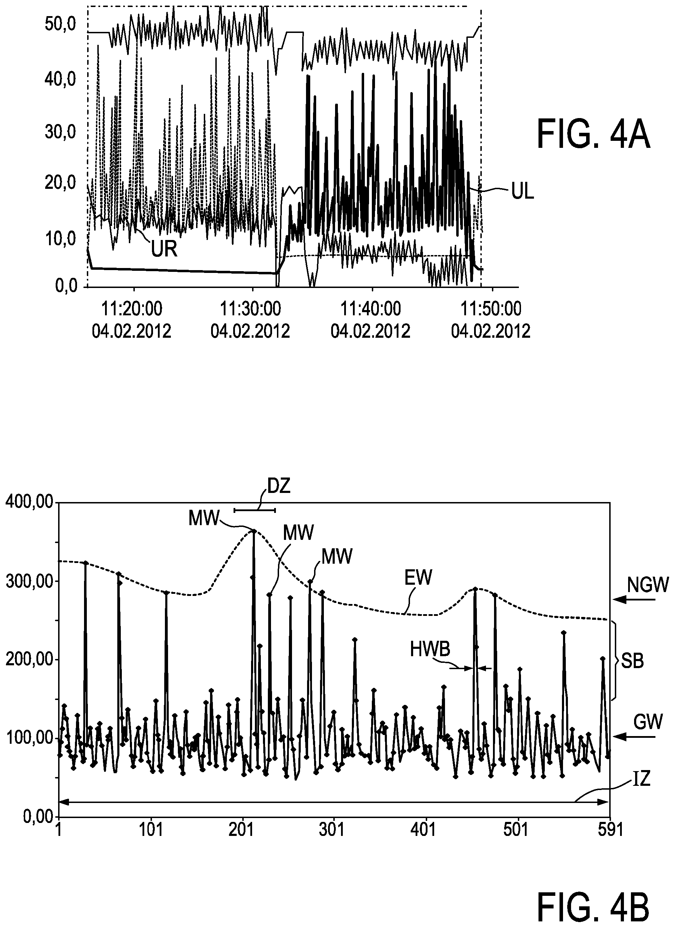

Fig. 4A    eine beispielhafte Abfolge von Zellspannungswerten der in Fig. 1 gezeigten Lambda-Sonden zusammen mit dem über die Nernst'sche Gleichung und Wasser-Gas-Gleichung zur Lambda-Sonde bestimmten Sauerstoff-Anteil bei einer Verbrennungssituation für den Fall einer ungenügenden Verbrennung mit der typischen Signatur von Zellspannungsspitzen;

Fig. 4B    eine vergrößerte Darstellung einer Abfolge von Zellspannungswerten der Fig. 4A auf gestreckter Zeitachse;

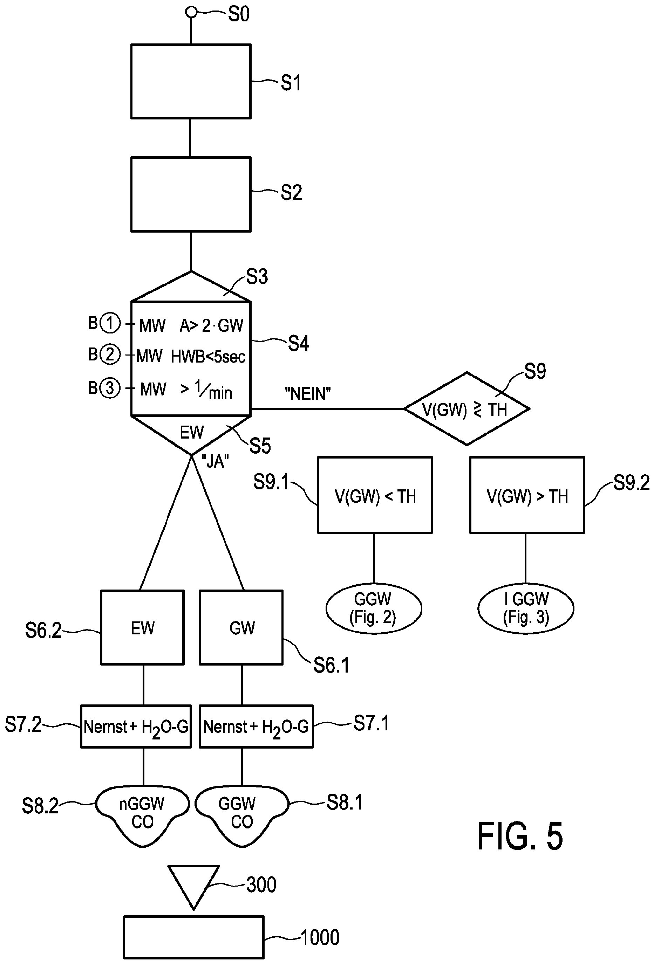

Fig. 5    ein Ablaufdiagramm eines Verfahrens zur Bestimmung eines Kohlenmonoxid-Anteils in einem Abgasstrom eines regenerativ befeuerten Industrieofens gemäß einer bevorzugten Ausführungsform.

Fig. 1 zeigt in vereinfachter Darstellung einen regenerativ beheizten Industrieofen 100 mit einem Ofenraum 10, dessen Oberofenraum 1 als Regelstrecke geregelt wird und bei dem der Unterofenraum 2 eine nicht näher dargestellte Glasschmelzwanne aufweist. In der Glasschmelzwanne enthaltenes Glas wird über den Ofenraum 10 über die Schmelztemperatur erwärmt und zur Herstellung von Flachglas oder dergleichen aufgeschmolzen und geeignet behandelt. Der Industrieofen 100 wird erwärmt, indem über mehrere seitlich angebrachte Brennstoffinjektoren 20 Brennstoff, vorliegend in Form von Brenngas, in den Oberofen 1 injiziert wird. Von den Brennstoffinjektoren 20 ist ein linker Injektor 20 dargestellt. Von weiteren Brennstoffinjektoren 20' ist ein rechter Injektor 20' dargestellt. Der Einfachheit halber sind im Folgenden für gleiche oder ähnliche Teile oder solche mit gleicher oder ähnlicher Funktion gleiche Bezugszeichen verwendet. Beispielsweise können linksseitig bzw. rechtsseitig jeweils eine Anzahl von sechs Injektoren 20, 20' vorgesehen sein. In der in Fig. 1 gezeigten Befeuerungsperiode wird über einen Brennstoffinjektor 20 Brenngas in den Oberofen 1 praktisch ohne Verbrennungsluft injiziert. Oberhalb des Brennstoffinjektors 20 wird vorgewärmte Verbrennungsluft VB über eine linksseitige Öffnung 30 dem Oberofen 1 zugeführt. Die Verbrennungsluft aus der Öffnung 30 vermischt sich im Oberofen 1 mit dem vom Brennstoffinjektor 20 injizierten Brenngas und führt zur Ausbildung einer den Unterofen überdeckenden Flamme 40, die vorliegend symbolisch dargestellt ist. Das Bild der Fig. 1 zeigt den Industrieofen 100 im Zustand einer regenerativen Befeuerung über den linken Regenerator 50 und die linken Injektoren 20. Diese und die Öffnung 30 sind derart gestaltet, dass das über die Injektoren 20 gelieferte Brenngas in ausreichendem nah- oder unter stöchiometrischen Bereich mit Verbrennungsluft des linken Regenerators im Oberofen 1 vermischt wird. Der in Fig. 1 dargestellte Betriebszustand einer linksseitigen Befeuerung des Oberofens 1 unter Injektion von Brenngas über die linksseitigen Injektoren 20 und Zufuhr von Verbrennungsluft VB über den linken Regenerator 50 dauert für eine erste Periodendauer an von z.B. 20 bis 40 min. an. Während dieser ersten Periodendauer wird Verbrennungsluft VB zum Oberofen 1 im Ofenraum 10 separat vom Brenngas 20 zugeführt. Während der ersten Periodendauer wird Abgas AG aus dem Oberofen 1 über rechtsseitige Öffnungen 30' dem rechten Regenerator 50' zugeführt und heizt diesen auf.

In einem zweiten Betriebszustand wird für eine zweite Periodendauer ähnlicher zeitlicher Länge die Befeuerung des Oberofens 1 umgekehrt. Dazu wird dann Verbrennungsluft VB über den rechten Regenerator 50' dem Oberofen 1 zusammen mit Brenngas aus den rechten Injektoren 20' zugeführt, wobei die Verbrennungsluft VB dann die vom Abgas AG in der ersten Periodendauer im Regenerator 50' deponierte Wärme aufnimmt.

Die Regelung eines Brennstoffstroms und/oder eines Verbrennungsluftstroms erfolgt grundsätzlich über ein Temperaturregelmodul 200 einer Steuereinrichtung 1000 für den Industrieofen 100. Grundsätzlich kann dazu ein PID-Regler im Temperaturregelmodul 200 eingesetzt werden, gemäß dem unter Erhöhung des Brennstoffstroms und/oder des Verbrennungsluftstroms eine Ofenraumtemperatur erhöht bzw. unter Erniedrigung eines Brennstoffstroms und/oder eines Verbrennungsluftstroms eine Ofenraumtemperatur erniedrigt wird. Dem Temperaturregelmodul 200 werden Temperaturwerte des Regeneratorkopfes 51 bzw. 51' oder des Oberofenraumes 1 über geeignete Sonden 52, 52', 53 zugeführt. Die Sonden 52, 52', 53 weisen vorliegend jedenfalls zum Teil neben einer Temperatursonde auch eine geeignete Lambdasonde zur Messung eines Brennstoff-Luft-Verhältnisses auf.

Insbesondere die über die Sonde 53 gemessene Temperatur im Oberofen dient als Eingang des Temperaturregelmoduls 200, z. B. um darauf basierend eine Temperaturmittelung und eine Extrapolation des Temperaturverhaltens auf das Ende einer Periodendauer vorzunehmen. Insbesondere die Temperaturen an einem Regeneratorkopf wie von den Sonden 52, 52' gemessen, können als Grundlage einer vereinfachten Ermittlung einer Vorwärmekenngröße dienen.

Die Sonden 52, 52' und auch die Sonde 53 bzw. gegebenenfalls an gleicher Stelle angeordnete Lambda-Sonden oder andere Messfühler können Messwerte, z. B. über Luft-oder Abgasmengen, für eine solche vereinfachte Ermittlung an den Eingang eines Lambdaauswertmoduls 300 der Steuereinrichtung 1000 liefern.

Fig. 2 zeigt den Verlauf von Zellspannungswerten einer Lambdasonde der Sonden 52, 52' für einen normalen Betrieb einer Glasschmelzwanne mit geringem aber ausreichend eingestelltem Sauerstoffüberschuss; d.h. bei Lambda-Werten zwischen 1,03 und 1,07. Die Restsauerstoff-Konzentration liegt dabei zwischen etwa 0,7 bis 1,5 % im Abgas. Konkret ist ein rechtsseitiger Verlauf von Zellspannungswerten UR als Funktion der Zeit mit zugeordnetem rechtsseitigem Restsauerstoff-Anteil OR gezeigt (das heißt bei linksseitiger Befeuerung). Für die darauffolgende Periodendauer sind linksseitige Zellspannungswerte UL mit zugeordnetem Restsauerstoff -Anteil OL gezeigt (das heißt bei rechtsseitiger Befeuerung). Die linksseitigen Zellspannungswerte UL liegen leicht unterhalb den rechtsseitigen Spannungswerten UR; entsprechend der Kennlinie der Lambda-Sonde --hier eine Zirkonoxid-Festelektrolytsonde-- liegen die Werte der linksseitigen Sauerstoff-Anteile OL über den Werten der rechtsseitigen Sauerstoff-Anteile OR. Die Restsauerstoff-Konzentrationen gemäß den Sauerstoff-Anteilen OL, OR liegen gemäß einer zweiten Berechnungsvorschrift auf Basis der Nernst'schen Gleichung zur Berechnung der $O_2$-Anteile in Prozent ($O_2$%)

$$--\ln(O_2\%) := 3.0417 - 46.4188 * U / (T+273)--$$

bei etwa zwischen 0,7 bis 1,5 %.

Die vergleichsweise geringen Konzentrationen für die Kohlenmonoxid-Anteile in % ($CO$%/$CO_2$%) im Gleich-gewichtsfall aus der Zellspannung U bestimmen sich mittels einer zweiten Berechnungsvorschrift auf Basis der Wasser-Gas-Gleichung als Verbrennungsvorschrift

$$--CO\%/CO_2\% = KW / EXP(((1374.5 - U)/(T+273) - 0.40399) / 0.043086).$$

Die Konzentrationen der Kohlenmonoxid-Anteile betragen etwa zwischen 50 bis 200 ppm. Vorzugsweise, aber nicht notwendigerweise ist T die Abgastemperatur statt der Temperatur der Lambda-Sonde.

Die Ermittlung eines Gleichgewichtswertes der Zellspannung --sei es die rechtsseitige oder die linksseitige Zellspannung UR, UL-- führt im Wesentlichen zu einer Konstante (als Funktion der Zeit jeweils für eine Periodendauer). Ein gegenüber dem Gleichgewichtswert erhöhter Nicht-Gleichgewichtswert lässt sich bei der in Fig. 2 dargestellten Situation nicht --jedenfalls nicht jenseits eines geeignet großen Schwellbereichs-- ermitteln. Ein geeignet großer Schwellbereich sollte einen Bereich von Zellspannungswerten umfassen, die zwischen einem 1.0-fachen des Gleichgewichtswerts und einem 1.5-fachen des Gleichgewichtswertes liegen. Bei der vorliegend dargestellten Situation eines Sauerstoff-Anteils bei normaler Verbrennungssituation --d.h. im Gleichgewicht und ausreichendem Sauerstoffangebot-- liegt ein gegenüber dem Gleichgewichtswert erhöhter Nicht-Gleichgewichtswert praktisch nicht vor. Die Zellspannungswerte liegen etwa pro Periodendauer im gleichen Bereich bzw. mit passabler Varianz um einen Gleichgewichtswert herum verteilt, wobei die Varianz deutlich unterhalb des genannten Schwellbereichs liegt.

Fig. 3 zeigt wiederum rechtsseitige Zellspannungswerte UR, die im Vergleich zu denen der Fig. 2 erhöht sind und zu einem entsprechend erniedrigten rechtsseitigen Sauerstoff-Anteil OR entsprechend einer Restsauerstoff-Konzentration führen. Bei der folgenden Periodendauer (rechtsseitige Befeuerung) liegen die linksseitigen Zellspannungswerte UL mit vergleichsweise starker Streuung um einen Mittelwert herum noch höher. Ein linksseitiger Sauerstoff-Anteil OL entspricht einer praktisch nicht vorhandenen Restsauerstoff-Konzentration. Ein Gleichgewichtswert lässt sich sowohl aus den rechtsseitigen Zellspannungswerten als auch aus den linksseitigen Zellspannungswerten UR, UL ermitteln, wenn auch bei den linksseitigen Zellspannungswerten UL mit vergleichsweise hoher Varianz. Allerdings erstreckt sich die Varianz unterhalb und oberhalb des gemeinsamen Gleichgewichtswerts. Ein gegenüber dem Gleichgewichtswert erhöhter Nicht-Gleichgewichtswert lässt sich über die Periodendauer bei rechtsseitiger Befeuerung und linksseitiger Befeuerung somit praktisch nicht angeben. Diese Situation ist bezeichnend für einen Betrieb des Industrieofens mit ungenügendem bzw. unterstöchiometrischem Luft-Anteil, das heißt bezeichnend für vollständig verbrannten Sauerstoff. Gleichwohl lassen sich wie erwähnt Gleichgewichtswerte als Mittelwerte über eine Periodendauer definieren und eine Gleichgewichtssituation trotz der Sauerstoffunterversorgung annehmen.

Fig. 4A zeigt dagegen beispielhaft eine Situation, auf der das Konzept der Erfindung beruht. Fig. 4A zeigt dazu zunächst rechtsseitige Zellspannungswerte UR (also bei linksseitiger Befeuerung) und für die folgende Periodendauer linksseitige Zellspannungswerte UL (also bei rechtsseitiger Befeuerung).

Rechnet man die vorgenannte erste Berechnungsvorschrift auf Basis der Nernst'schen Gleichung und die vorgenannte zweite Berechnungsvorschrift auf Basis der Wasser-Gas-Gleichung durch, ergibt sich eine Kohlenmonoxid-Konzentration, die nicht nur geringfügig über der Gleichgewichts-Konzentration eines Kohlenmonoxid-Anteils liegt, sondern es können in einem solchen Fall 20- bis 50-fach oder noch weit höhere Konzentrationen entsprechend einem sehr stark erhöhten Kohlenmonoxid-Anteil auftreten. Dies zeigt sich durch die auffälligen, nach oben --also nur einseitig in einen positiven Bereichausgebildeten Zellspannungsspitzen mit Maximalwerten MW der Zellspannungswerte im Vergleich zu einem erkennbaren Gleichgewichtswert GW.

Überraschend wurde gefunden, dass diese Signatur charakteristisch für eine mangelnde Verbrennungsdynamik mit überhöhter Kohlenmonoxid-Konzentration ist, welche in den Zellspannungswerten die wiederkehrenden Zellspannungsspitzen mit zeitlich begrenzten Maximalwerten MW verursacht. In der Tat werden diese an der an sich zur Sauerstoffmessung bestimmten Zirkonoxidsonde erkennbar und messbar. Die an der Zirkonoxid-sonde --nämlich Sonde 52, 52'-- messbaren Zellspannungswerte entsprechen weitaus überwiegend der Sauerstoff-Konzentration im Abgasstrom. Auswertbar sind diese , wie oben angegeben, mit der ersten Berechnungsvorschrift auf Basis der Nernst'schen Gleichung. Der Messdatenstrom an Zellspannungswerten wird jedoch auch durch die bisher unberücksichtigt gebliebenen, sehr kurzzeitige Zellspannungsspitzen überlagert, die der über das chemische Gleichgewicht hinaus deutlich erhöhten Kohlenmonoxid-Konzentration entsprechen.

Genauere Beobachtungen --wie sie ausschnittsweise in Fig. 4B aus dem in Fig. 4A gezeigten Zellspannungswerten herausgenommen sind-- auf getreckter Zeitskala zeigen, dass solche Zellspannungsspitzen auf 1 bis 3 Sekunden Halbwertsbreite HWB begrenzt sind. Das heißt, bei einer herkömmlichen Signalauswertung der Zirkonoxidsonde gehen diese im Rauschen unter bzw. werden durch eine Mittelwertbildung bei Ermittlung des Gleichgewichtswertes GW unterdrückt.

Es wurde erkannt, dass dies -- um nur mit einer einfachen Mittelwertbildung einen Kohlenmonoxid-Anteil zu bestimmen-- in Anbetracht einer Signatur, wie sie in Fig. 4B erkennbar ist, nicht ausreichend ist. Vielmehr lassen sich zwei separate Signalsignaturen erkennen --nämlich eine überwiegende Vielzahl von Messwerten, die um einen Gleichgewichtswert GW herum verteilt sind und die erwähnte geringere Vielzahl von Messwerten, die um einen erhöhten Nicht-Gleichgewichtswert NGW herum verteilt sind.

Der Gleichgewichtswert GW lässt sich auf einem in Fig. 4B gezeigten Integralzeitfenster IZ durch Mittelung aller dort existierenden Zellspannungsmesswerte ermitteln. Der Gleichgewichtswert GW liegt hier etwa bei 100 mV.

Fig. 4B zeigt einen Fall mangelhafter Verbrennungsdynamik, bei dem trotz ausreichender Sauerstoffrest-Konzentration von über 1,5 %, eine kritische Konzentration von Kohlenmonoxid von mehr als 2000 bis 5000 ppm beobachtet wird; das heißt Konzentrationen, die 20- bis 50-fach über einer Konzentration eines theoretischen Gleichgewichts-Kohlenmonoxid-Anteils liegen. Derartige Über-Konzentrationen an Kohlenmonoxid CO über dem Gleichgewichtszustand gehen typsicherweise auch mit überhöhten Abgastemperaturen einher. Es werden Temperaturerhöhungen bis zu 50 K gegenüber normalen Ausbrandverhältnissen beobachtet. Kohlenmonoxid (CO) selbst --ebenso wie die genannten Übertemperaturen-- verursachen vermeidbare Energieverluste und drohen das feuerfeste Material des Ofens zu beschädigen. Im vorliegenden Fall lässt sich in der Tat ein gegenüber dem Gleichgewichtswert erhöhter Nicht-Gleichgewichtswert NGW ermitteln, wobei der erhöhte Nicht-Gleichgewichtswert NGW zur Ermittlung eines zweiten, einen Nicht-Gleichgewichts-Anteil kennzeichnenden Kohlenmonoxid-Anteils im Abgas genutzt wird.

Der erhöhte Nicht-Gleichgewichtswert NGW wird vorliegend als Mittelwert aller Zellspannungswerte oberhalb eines Schwellbereichs und in einem Differential-Zeitfenster DZ ermittelt. Das Integral-Zeitfenster IZ beträgt vorliegend etwa 600 Sekunden; also in etwa 10 Minuten. Das beispielhaft eingezeichnete Differential-Zeitfenster DZ beträgt vorliegend nur etwa 10 Sekunden. Fährt man das Differential-Zeitfenster DZ durch das Integral-Zeitfenster IZ und bildet über das Differential-Zeitfenster einen laufenden Mittelwert von Zellspannungswerten oberhalb eines Schwellbereichs SB --der vorliegend zwischen 150 und 250 mV liegt-- ergibt sich der in Fig. 4B schematisch eingezeichnete Verlauf eines Enveloppenwerts EW als Funktion der Zeit. Vorliegend ergibt sich ein Enveloppenwert EW als Funktion der Zeit über eine laufende Kurzzeit-Mittelwertbildung von Zellspannungswerten über das Differential -Zeitfenster soweit die Zellspannungswerte oberhalb des Schwellbereichs SB liegen. Auch liegt ein gegenüber dem Gleichgewichtswert erhöhter Nicht-Gleichgewichtswert NGW als Funktion der Zeit vor, und kann als laufender Kurzzeit-Mittelwert oberhalb eines Schwellbereichs gebildet sein. Verringert man den Schwellbereich SB auf einen Bereich von 150 bis 200 mV, würde der Enveloppenwert EW stärker schwanken. Er würde sich insbesondere bei weiterer Verkleinerung des Differential-Zeitfensters DZ noch weiter auf die Zellspannungsspitzen konzentrieren; d.h. mit größerer Amplitudenschwankung und geringerer Halbwertsbreite HWB um einen Maximalwert MW herum.

Grundsätzlich kann zur Identifizierung der Zellspannungsspitzen eine einstellbare große Zahl von Zellspannungswerten für ein Integral-Zeitfenster IZ in einer elektronischen Auswertevorrichtung gleitend gespeichert werden, wobei ein Maximalwert MW der gespeicherten Zellspannungswerte --vorliegend als leicht gemittelter Enveloppenwert EWzur Bestimmung eines aktuellen Kohlenmonoxid-Anteils genutzt wird. Dazu zeigt Fig. 5 ein Ablaufschema für eine besonders bevorzugte Ausführung des Verfahrens zur Bestimmung eines Kohlenmonoxid-Anteils in einem Abgasstrom. Das Verfahren ist geeignet, Zellspannungswerte, wie sie in Fig. 4A und Fig. 4B gezeigt sind, zu behandeln und zu diskriminieren, vom normalen Gleichgewichtsszenario mit guter Verbrennung, wie dies in Fig. 2 gezeigt ist und zu diskriminieren, vom Gleichgewichtsszenario mit Sauerstoffunterversorgung, wie dies in Fig. 3 gezeigt ist.

Dazu ist in der bevorzugten Ausführungsform vorgesehen, dass in einem Startschritt S0 zunächst eine Kalibrierung der Anlage vorgesehen ist. Zur Kalibrierung kann in einer Kohlenmonoxid-Messung --im Vergleich mit einer anderen, unabhängigen Kohlenmonoxid-Konzentrationsmessung am gleichen Messort-- eine Einstellung der einstellbaren Anzahl von Zellspannungswerten variiert werden. Es kann zusätzlich oder alternativ eine Annahme einer Temperaturdifferenz zwischen analysiertem Abgas AG und der Temperatur der Sonde 52, 52' oder 53 selbst variiert werden.

In einem ersten Verfahrensschritt S1 werden Zellspannungswerte --einer Lambda-Sonde z.B. der Sonden 52, 52'-- gemessen, beispielsweise über mehrere Periodendauern, wie dies in Fig. 2 bis Fig. 3 gezeigt ist. In einem zweiten Verfahrensschritt S2 wird ein Gleichgewichtswert aus der Zellspannung ermittelt, indem für ein Integral-Zeitfenster IZ alle Zellspannungswerte gemittelt werden und der Mittelwert als Gleichgewichtswert GW angenommen wird. Diese Vorgehensweise führt in einer Gleichgewichtssituation bei Normalbetrieb (Fig. 2) zu einem Gleichgewichtswert GW mit geringer Varianz und in einem Betrieb unter Gleichgewicht mit Sauerstoffunterversorgung (Fig. 3) zu einem Gleichgewichtswert GW mit vergleichsweise großer Varianz. Dies gilt grundsätzlich auch für die Situation der Fig. 4A und Fig. 4B. Jedoch zeigt sich, dass hier eine hohe Fluktuation von Spannungswerten zum einen vermehrt auftritt und zum anderen ist deren Amplitude nicht betragsmäßig um einen Gleichgewichtswert GW gleichmäßig verteilt, d.h. nach oben und unten mit gleichem Ausschlag, sondern überwiegend einseitig nach oben verschoben.

In einem dritten Schritt S3 wird geprüft, ob eine Situation, wie sie in Fig. 4A und Fig. 4B gezeigt ist, vorliegt. Dazu wird geprüft, ob Zellspannungswerte als ausgeprägte deutlich amplitudenstärkere Zellspannungsspitzen vorliegen. Dazu können in einem vierten Schritt S4 die dort aufgeführten drei Bedingungen B1, B2, B3 überprüft werden. Dazu wird geprüft, ob in einer ersten Bedingung B1 Zellspannungsspitzen ausschließlich oberhalb eines Gleichgewichtswertes mit einer Amplitude A > 2*GW vorliegen. Auch kann in einer zweiten Bedingung B2 für einen Maximalwert MW geprüft werden, ob dessen Halbwertsbreite HWB im Mittel unterhalb von 5 Sekunden liegt. Als dritte Bedingung B3 kann geprüft werden, ob die Maximalwerte MW mit einer Häufigkeit # auftreten, die größer ist als eine Zellspannungsspitze pro Minute.

Liegen die drei Bedingungen B1, B2 und B3 nicht kumulativ vor, kann in der Abzweigung "NEIN" in einem neunten Schritt S9 geprüft werden, ob eine Varianz V (GW) des Gleichgewichtswertes GW oberhalb oder unterhalb eines Threshold TH liegt.

Liegt die Varianz des Gleichgewichtswertes V(GW) in einem ersten des neunten Schrittes S9.1 unterhalb eines Threshold TH, kann auf eine Gleichgewichtssituation GGW bei guten Verbrennungsbedingungen, wie sie in Fig. 2 dargestellt ist, geschlossen werden.

Liegt die Varianz des Gleichgewichtswertes V(GW) oberhalb eines Threshold TH in einem zweiten des neunten Schrittes S9.2, kann auf eine labile Gleichgewichtssituation IGGW, gegebenenfalls mit Sauerstoffunterversorgung gemäß Fig. 3 geschlossen werden.

Liegen die drei Bedingungen B1 bis B3 kumulativ vor, kann entlang des "JA"-Zweigs in einem fünften Schritt S5 beispielsweise ein Enveloppenwert EW zu den Maximalwerten MW bestimmt werden. In einem ersten des sechsten Schrittes S6.1 kann dann der Gleichgewichtswert GW als Funktion der Zeit zur Verfügung gestellt werden. In einem ersten des siebten Schrittes S7.1 kann der Gleichgewichtswert GW mit Hilfe der ersten Berechnungsvorschrift auf Basis der Nernst'schen Gleichung und der zweiten Berechnungsvorschrift auf Basis der Wasser-Gas-Gleichung genutzt werden, um eine Gleichgewichts-Kohlenmonoxid-Konzentration GGW-CO in einem ersten des achten Schrittes S8.1 zu bestimmen.

Ebenso kann in einem weiteren sechsten Schritt S6.2 der Enveloppenwert EW zur Verfügung gestellt werden als Funktion der Zeit. Dieser Enveloppenwert EW kann in einem weiteren siebten Schritt S7.2 der ersten Berechnungsvorschrift auf Basis der Nernst'schen Gleichung und der zweiten Berechnungsvorschrift auf Basis der Wasser-Gas-Gleichung unterworfen werden, um in einem weiteren achten Schritt S8.2 einen Nicht-Gleichgewichts-Anteil eines Kohlenmonoxid-Anteils nGGW-CO anzugeben. Die Ergebnisse eines Nicht-Gleichgewichts-Kohlenmonoxid-Anteils nGGW-CO und eines Gleichgewichts-Kohlenmonoxid-Anteils GGW-CO können einem Betriebsverfahren im Lambdawert-Modul 300 zugeführt werden, das beispielsweise auf einer Steuereinrichtung 1000 des Industrieofens 100 der Fig. 1 ausgeführt wird.

Bezugszeichenliste

| | |
|---|---|
| 1 | Oberofenraum |
| 2 | Unterofenraum |
| 10 | Ofenraum |
| 20 | Brenngas |
| 20 | linker Brennstoffinjektor |
| 20' | rechter Brennstoffinjektor |
| 30 | linksseitige Öffnung |
| 30' | rechtsseitige Öffnung |
| 40 | Flamme |
| 50 | Regenerator |
| 50' | rechter Regenerator |
| 51 | Regeneratorkopf |
| 51' | Regeneratorkopf |
| 52 | Sonde |
| 52' | Sonde |
| 53 | Sonde |
| 100 | Industrieofen |
| 200 | Temperaturregelmodul |
| 300 | Lambdawertmodul |
| 1000 | Steuereinrichtung |

| | |
|---|---|
| AG | Abgas |
| B1, B2, B3 | Bedingungen |
| CO | Kohlenmonoxid |
| $CO_2$ | Kohlendioxid |
| DZ | Differenzialzeitfenster |
| EW | Enveloppenwert |
| GW | Gleichgewichtswert |
| GGW | Gleichgewichtssituation |
| GGW-CO | Gleichgewichtskonzentration Kohlenmonoxid |
| HWB | Halbwertbreite |
| IZ | Integralzeitfenster |

IGGW          labile Gleichgewichtssituation
MW          Maximalwert
NGW         Nicht-Gleichgewichtswert
nGGW-CO     Nicht-Gleichgewichtsanteil Kohlenmonoxid
OL           linksseitiger Sauerstoff-Anteil
OR          rechtsseitiger Sauerstoff-Anteil
$O_2$           Sauerstoff
UL           linksseitiger Verlauf von Zellspannungswerten
UR          rechtsseitiger Verlauf von Zellspannungswerten
VB          vorgewärmte Verbrennungsluft

**Patentansprüche**

1. Verfahren zur Bestimmung eines Kohlenmonoxid-Anteils in einem Abgasstrom eines regenerativ befeuerten Industrieofens (100), insbesondere eines Glasschmelzofens, für ein Verfahren zur Steuerung und/oder Regelung des regenerativ befeuerten Industrieofens, **dadurch gekennzeichnet, dass**:

   - in dem Abgasstrom eine Lambda-Sonde (52,52') eingebracht ist, und eine Temperatur der Lambdasonde zur Verfügung gestellt wird, und eine erste Berechnungsvorschrift eine Sondenvorschrift ist, welche die Zellspannung und die Temperatur der Lambda-Sonde nutzt auf Basis der Nernst'schen Gleichung und mittels der ersten Berechnungsvorschrift ein Sauerstoff-Anteil im Abgas ermittelt wird auf Basis einer Nernst'schen Gleichung, und
   - wenigstens eine Zellspannung mittels Zellspannungswerten (UL,UR) als Funktion der Zeit zur Verfügung gestellt wird, auf deren Grundlage
   - mittels einer zweiten Berechnungsvorschrift ein Kohlenmonoxid-Anteil im Abgas ermittelt wird, und eine Abgas-Temperatur und eine Brennstoff-Zusammensetzung zur Verfügung gestellt wird, und die zweite Berechnungsvorschrift für einen Kohlenmonoxid-Anteil im Abgas eine Verbrennungsvorschrift ist, welche die Brennstoff-Zusammensetzung nutzt auf Basis einer Wasser-Gas-Gleichung, wobei

   aus den Zellspannungswerten

   - wenigstens ein Gleichgewichtswert (GW) ermittelt wird und
   - wenigstens ein gegenüber dem Gleichgewichtswert erhöhter Nicht-Gleichgewichtswert (NGW) ermittelt wird, wobei

   der Gleichgewichtswert zur Ermittlung eines ersten, einen Gleichgewichts-Anteil kennzeichnenden Kohlenmonoxid-Anteils im Abgas genutzt wird, und
   der erhöhte Nicht-Gleichgewichtswert zur Ermittlung eines zweiten, einen Nichtgleichgewichts-Anteil kennzeichnenden, Kohlenmonoxid-Anteils im Abgas genutzt wird, und

   - aus der Zellspannung der gegenüber dem Gleichgewichtswert erhöhte Nicht-Gleichgewichtswert oberhalb eines Schwellbereichs (SB) als Funktion der Zeit aus Zellspannungsspitzen der Zellspannungswerte ermittelt wird, und
   - zur Identifizierung der Zellspannungsspitzen eine einstellbare große Anzahl von Zellspannungswerten für ein Integralzeitfenster (IZ) in einer Signalauswertung gleitend gespeichert wird, wobei ein Maximum (MW) oder ein Enveloppenwert (EW) der gespeicherten Zellspannungswerte zur Bestimmung des aktuellen zweiten, den Nichtgleichgewichts-Anteil kennzeichnenden, Kohlenmonoxid-Anteils im Abgas genutzt wird.

2. Verfahren nach Anspruch 1 **dadurch gekennzeichnet, dass** die zweite Berechnungsvorschrift zur Ermittlung des zweiten, einen Nichtgleichgewichts-Anteil kennzeichnenden, Kohlenmonoxid-Anteils, einen Sauerstoff-Anteil im Abgas vernachlässigt oder unter Nutzung eines geringen bis vernachlässigbaren Sauerstoff-Anteils den Nichtgleichgewichts-Anteil von Kohlenmonoxid ermittelbar macht.

3. Verfahren nach Anspruch 1 oder 2 **dadurch gekennzeichnet, dass** zur Ermittlung des ersten, einen Gleichgewichts-Anteil kennzeichnenden, Kohlenmonoxid-Anteils im Abgas

   - mittels einer ersten Berechnungsvorschrift ein Sauerstoff-Anteil im Abgas ermittelt wird, und unter Nutzung eines nicht vernachlässigbaren Sauerstoff-Anteils im Abgas, und

- unter Nutzung des genannten Gleichgewichtswert mittels der zweiten Berechnungsvorschrift ein Kohlenmonoxid-Anteil im Abgas ermittelt wird, welcher einen Gleichgewichts-Anteil kennzeichnenden Kohlenmonoxid-Anteil im Abgas kennzeichnet.

4. Verfahren nach einem der Ansprüche 1 bis 3 **dadurch gekennzeichnet, dass** für die Zellspannung das Integralzeitfenster als ein bestimmtes Integralzeitfenster umfassend eine erste Anzahl von Zellspannungswerten und/oder ein bestimmtes Differentialzeitfenster umfassend eine zweite Anzahl von Zellspannungswerten vorgegeben wird, wobei das Differentialzeitfenster weniger Zellspannungswerte umfasst als das Integralzeitfenster.

5. Verfahren nach Anspruch 1 oder 4 **dadurch gekennzeichnet, dass** der Gleichgewichtswert als Mittelwert aller Zellspannungswerte im Integralzeitfenster ermittelt wird und/oder der erhöhte Nicht-Gleichgewichtswert als der Mittelwert oder der Enveloppenwert aller Zellspannungswerte oberhalb des Schwellbereichs und im Differentialzeitfenster ermittelt wird.

6. Verfahren nach einem der Ansprüche 1 bis 5, **dadurch gekennzeichnet, dass** die Signalauswertung aus den Zellspannungswerten der Lambdasonde die Zellspannungsspitzen der Zellspannungswerte separiert.

7. Verfahren nach einem der Ansprüche 1 bis 6 **dadurch gekennzeichnet, dass** aus der Zellspannung

- der Gleichgewichtswert unterhalb des Schwellbereichs als Funktion der Zeit ermittelt wird.

8. Verfahren nach einem der Ansprüche 1 bis 7 **dadurch gekennzeichnet, dass** nur solche Zellspannungswerte zur Bestimmung des erhöhten Nicht-Gleichgewichtswerts genutzt werden, die den Gleichgewichtswert um einen Faktor von gleich oder mehr als 1.5 übersteigen.

9. Verfahren nach Anspruch 8 **dadurch gekennzeichnet, dass** der Schwellbereich einen Bereich von Zellspannungswerten umfasst, die zwischen einem 1.0-fachen des Gleichgewichtswerts und einem 1.5-fachen des Gleichgewichtswertes liegen.

10. Verfahren nach einem der Ansprüche 1 bis 9 **dadurch gekennzeichnet, dass** nur solche Zellspannungswerte zur Bestimmung des erhöhten Nicht-Gleichgewichtswerts genutzt werden, die eine Halbwertsbreite unterhalb von 10 Sekunden haben und ein Differentialzeitfenster eine vorzugsweise einstellbare Breite zwischen 2 Sekunden und 100 Sekunden hat.

11. Verfahren nach einem der Ansprüche 1 bis 10 **dadurch gekennzeichnet, dass** zur Bestimmung des erhöhten Nicht-Gleichgewichtswerts heranzuziehende Zellspannungswerte mit einer durchschnittlichen Rate von einem bis fünf Werten pro Minute auftreten, wobei das Integralzeitfenster derart gewählt ist, dass wenigstens im Mittel zwei zur Bestimmung des erhöhten Nicht-Gleichgewichtswerts heranzuziehende Zellspannungswerte im Integralzeitfenster auftreten, wobei das Integralzeitfenster in einem Bereich oberhalb von 100 Sekunden liegt.

12. Verfahren nach einem der Ansprüche 1 bis 11 **dadurch gekennzeichnet, dass** der erste Kohlenmonoxid-Anteil im Abgas aus dem Gleichgewichtswert und der zweite Kohlenmonoxid-Anteil im Abgas aus dem erhöhten Nicht-Gleichgewichtswert mittels der gleichen wenigstens einen zweiten Berechnungsvorschrift ermittelt wird, wobei eine Anwendung einer Nernst'schen Gleichung und eine Anwendung einer Wasser-Gas-Gleichung für eine Bestimmung des ersten Kohlenmonoxid-Anteils im Abgas aus dem Gleichgewichtswert, optional unter Berücksichtigung eines nicht vernachlässigbaren Sauerstoff-Anteils, auf dieselbe Weise erfolgt, wie für eine Bestimmung des zweiten Kohlenmonoxid-Anteils, optional ohne Berücksichtigung eines vernachlässigbaren Sauerstoff-Anteils.

13. Verfahren nach einem der Ansprüche 1 bis 12 **dadurch gekennzeichnet, dass** der erhöhte Nicht-Gleichgewichtswert zur Ermittlung des zweiten, einen Nichtgleichgewichts-Anteil kennzeichnenden, Kohlenmonoxid-Anteils, im Abgas nur dann für eine Verfahren zur Steuerung und/oder Regelung eines regenerativ befeuerten Industrieofens genutzt wird, wenn der erhöhte Nicht-Gleichgewichtswert einen Schwellbereich übersteigt.

14. Verfahren nach einem der Ansprüche 1 bis 13 **dadurch gekennzeichnet, dass** der erhöhte Nicht-Gleichgewichtswert zur Ermittlung des zweiten, einen Nichtgleichgewichts-Anteil kennzeichnenden, Kohlenmonoxid-Anteils, im Abgas nur dann für eine Verfahren zur Steuerung und/oder Regelung eines regenerativ befeuerten Industrieofens genutzt wird, wenn eine Abgastemperatur wenigstens 10K oberhalb einer Normaltemperatur liegt.

**15.** Verfahren nach einem der Ansprüche 1 bis 14 **dadurch gekennzeichnet, dass** zur Kalibrierung einer Kohlenmonoxid-Messung im Vergleich mit einer anderen, unabhängigen Kohlenmonoxid-Konzentrationsmessung am gleichen Messort eine Einstellung der einstellbaren Anzahl von Zellspannungswerten variierbar verwendet wird für das Integral-zeitfenster zur gleitenden Speicherung in der Signalauswertung, insbesondere einer elektronischen Auswerteeinheit.

**16.** Verfahren nach einem der Ansprüche 1 bis 15 **dadurch gekennzeichnet, dass** eine Annahme einer Temperaturdifferenz zwischen einer Abgas-Temperatur des analysiertem Abgases und der Temperatur der Lambda-Sonde selbst variierbar verwendet wird, nämlich einerseits die Temperatur der Lambda-Sonde zur Verwendung in der ersten Berechnungsvorschrift, welche die Zellspannung und die Temperatur der Lambda-Sonde nutzt auf Basis einer Nernst'schen Gleichung, und andererseits die Abgas-Temperatur zur Verwendung in der zweiten Berechnungsvorschrift, welche die welche die Brennstoff-Zusammensetzung nutzt auf Basis einer Wasser-Gas-Gleichung.

**17.** Steuereinrichtung (1000) für einen regenerativ befeuerten Industrieofen (100), insbesondere für einen Glasschmelzofen, ausgebildet zur Ausführung des Verfahrens nach einem Ansprüche 1 bis 16.

**18.** Regenerativ befeuerter Industrieofen (100), insbesondere Glasschmelzofen, mit der Steuereinrichtung nach Anspruch 17.

**Claims**

**1.** Method for determining a carbon monoxide fraction in an exhaust gas stream of a regeneratively fired industrial furnace (100), in particular a glass furnace, for a method for controlling and/or regulating the regeneratively fired industrial furnace, **characterised in that**:

- a lambda probe (52, 52') is introduced into the exhaust gas stream, and a temperature of the lambda probe is provided, and a first calculation rule is a probe rule which uses the cell voltage and the temperature of the lambda probe on the basis of the Nernst equation, and by means of the first calculation rule an oxygen fraction in the exhaust gas is determined on the basis of a Nernst equation, and
- at least one cell voltage is provided by means of cell voltage values (UL, UR) as a function of time, on the basis of which
- by means of a second calculation rule a carbon monoxide fraction in the exhaust gas is determined, and an exhaust gas temperature and a fuel composition are provided, and the second calculation rule for a carbon monoxide fraction in the exhaust gas is a combustion rule which uses the fuel composition on the basis of a water-gas equation, whereby from the cell voltage values
- at least one equilibrium value (GW) is determined and
- at least one non-equilibrium value (NGW) which is increased compared with the equilibrium value is determined, whereby the equilibrium value is used for determination of a first carbon monoxide fraction in the exhaust gas which characterises an equilibrium fraction, and the increased non-equilibrium value is used for determination of a second carbon monoxide fraction which characterises a non-equilibrium fraction, and
- from the cell voltage the non-equilibrium value which is increased compared with the equilibrium value is determined above a threshold range (SB) as a function of time from cell voltage peaks of the cell voltage values, and
- for identification of the cell voltage peaks an adjustable large number of cell voltage values for an integral time window (IZ) in a signal evaluation are stored in a moving manner, whereby a maximum (MW) or an envelope value (EW) of the stored cell voltage values is used for determination of the current second carbon monoxide fraction in the exhaust gas which characterises the non-equilibrium fraction.

**2.** Method according to claim 1, **characterised in that**, for determination of the second carbon monoxide fraction which characterises a non-equilibrium fraction, the second calculation rule ignores an oxygen fraction in the exhaust gas or, by using a minimal to insignificant oxygen fraction, makes it possible to determine the non-equilibrium fraction of carbon monoxide.

**3.** Method according to claim 1 or 2, **characterised in that**, for determination of the first carbon monoxide fraction in the exhaust gas which characterises an equilibrium fraction,

- by means of a first calculation rule an oxygen fraction in the exhaust gas is determined, and by using a significant oxygen fraction in the exhaust gas, and
- by using the said equilibrium value by means of the second calculation rule a carbon monoxide fraction in the exhaust gas is determined, which characterises a carbon monoxide fraction in the exhaust gas which characterises an equilibrium fraction.

4.   Method according to any of claims 1 to 3, **characterised in that** for the cell voltage the integral time window is predetermined as a specific integral time window comprising a first number of cell voltage values and/or a specific differential time window comprising a second number of cell voltage values, whereby the differential time window comprises fewer cell voltage values than the integral time window.

5.   Method according to claim 1 or 4, **characterised in that** the equilibrium value is determined as the average value of all cell voltage values in the integral time window and/or the increased non-equilibrium value is determined as the average value or the envelope value of all cell voltage values above the threshold range and in the differential time window.

6.   Method according to any of claims 1 to 5, **characterised in that** the signal evaluation separates the cell voltage peaks of the cell voltage values out from the cell voltage values of the lambda probe.

7.   Method according to any of claims 1 to 6, **characterised in that** from the cell voltage

- the equilibrium value below the threshold range is determined as a function of time.

8.   Method according to any of claims 1 to 7, **characterised in that** for determination of the increased non-equilibrium value only those cell voltage values are used which exceed the equilibrium value by a factor of equal to or more than 1.5.

9.   Method according to claim 8, **characterised in that** the threshold range comprises a range of cell voltage values which lie between 1.0 times the equilibrium value and 1.5 times the equilibrium value.

10.  Method according to any of claims 1 to 9, **characterised in that** for determination of the increased non-equilibrium value only those cell voltage values which have a half width value below 10 seconds are used, and a differential time window has a preferably adjustable width between 2 seconds and 100 seconds.

11.  Method according to any of claims 1 to 10, **characterised in that** cell voltage values used for determination of the increased non-equilibrium value occur at an average rate of one to five values per minute, whereby the integral time window is selected in such a way that at least on average two cell voltage values to be used for determination of the raised non-equilibrium value occur in the integral time window, whereby the integral time window is in a range above 100 seconds.

12.  Method according to any of claims 1 to 11, **characterised in that** the first carbon monoxide fraction in the exhaust gas is determined from the equilibrium value and the second carbon monoxide fraction in the exhaust gas is determined from the increased non-equilibrium value by means of the same at least one second calculation rule, whereby an application of a Nernst equation and/or an application of a water-gas equation for determination of the first carbon monoxide fraction in the exhaust gas from the equilibrium value, optionally taking account of a significant oxygen fraction, takes place in the same way as for a determination of the second carbon monoxide fraction, optionally without taking account of an insignificant oxygen fraction.

13.  Method according to any of claims 1 to 12, **characterized in that** the increased non-equilibrium value for determination of the second carbon monoxide fraction which characterises a non-equilibrium fraction in the exhaust gas is used for a method for controlling and/or regulating a regeneratively fired industrial furnace only when the increased non-equilibrium value exceeds a threshold range.

14.  Method according to any of claims 1 to 13, **characterised in that** the increased non-equilibrium value for determination of a second carbon monoxide fraction which characterises a non-equilibrium fraction in the exhaust gas is only used for a method for controlling and/or regulating a regeneratively fired industrial furnace when an exhaust gas temperature is at least 10K above the normal temperature.

15.  Method according to any of claims 1 to 14, **characterised in that**, for calibration of a carbon monoxide measurement by comparison with another independent carbon monoxide concentration measurement at the same measurement location, an adjustment of the adjustable number of cell voltage values is used variably for the integral time window for moving storage in the signal evaluation, in particular in an electronic evaluation unit.

16. Method according to any of claims 1 to 15, **characterised in that** an assumption of a temperature difference between an exhaust gas temperature of the analysed exhaust gas and the temperature of the lambda probe itself is used variably, that is to say on the one hand the temperature of the lambda probe for use in the first calculation rule, which uses the cell voltage and the temperature of the lambda probe on the basis of a Nernst equation, and on the other hand the exhaust gas temperature for use in the second calculation rule, which uses the fuel composition on the basis of a water-gas equation.

17. Control device (1000) for a regeneratively fired industrial furnace (100), in particular for a glass furnace, designed to carry out the method according to any of claims 1 to 16.

18. Regeneratively fired industrial furnace (100), in particular glass furnace having the control device according to claim 17.

**Revendications**

1. Procédé de détermination d'un taux de monoxyde de carbone dans un flux de gaz d'échappement d'un four industriel régénératif (100), plus particulièrement d'un four de fusion de verre, pour un procédé de commande et/ou de régulation du four industriel régénératif, **caractérisé en ce que** :

   - dans le flux de gaz d'échappement est intégrée une sonde lambda (52, 52') et une température de la sonde lambda est mise à disposition et
   - une première règle de calcul est une règle de sonde qui utilise la tension de cellule et la température de la sonde lambda sur la base de l'équation de Nernst et, au moyen de la règle de calcul, un taux d'oxygène dans le gaz d'échappement est déterminé sur la base de l'équation de Nernst et
   - au moins une tension de cellule est mise à disposition au moyen de valeurs de tension de cellule (UL, UR) en fonction du temps, sur la base de laquelle
   - au moyen d'une deuxième règle de calcul, un taux de monoxyde de carbone dans le gaz d'échappement est déterminé et une température de gaz d'échappement et une composition du carburant est mise à disposition et la deuxième règle de calcul pour un taux de monoxyde de carbone dans le gaz d'échappement est une règle de combustion qui utilise la composition du carburant sur la base d'une équation eau-gaz, dans lequel, à partir des valeurs de tension de cellule,
   - au moins une valeur d'équilibre (GW) est déterminée et
   - au moins une valeur de non-équilibre (NGW), augmentée par rapport à la valeur d'équilibre, est déterminée, dans lequel la valeur d'équilibre est utilisée pour la détermination d'un premier taux de monoxyde de carbone dans le gaz d'échappement caractérisant un taux d'équilibre et la valeur de non-équilibre augmentée est utilisée pour la détermination d'un deuxième taux de monoxyde de carbone caractérisant un taux de non-équilibre et
   - à partir de la tension de cellule, la valeur de non-équilibre augmentée par rapport à la valeur d'équilibre est déterminée au-dessus d'une zone seuil (SB) en fonction du temps à partir des pointes de tension de cellule des valeurs de tension de cellule et
   - pour l'identification des pointes de tension de cellule, un grand nombre réglable de valeurs de tension de cellule pour une fenêtre de temps d'intégrale (IZ) sont enregistrées de manière glissante dans une analyse de signaux, dans lequel un maximum (MW) ou une valeur d'enveloppe (EW) des valeurs de tension de cellule est utilisée pour la détermination du deuxième taux de monoxyde de carbone dans le gaz d'échappement actuel caractérisant la valeur de non-équilibre.

2. Procédé selon la revendication 1, **caractérisé en ce que** la deuxième règle de calcul pour la détermination du deuxième taux de monoxyde de carbone caractérisant un taux de non-équilibre néglige un taux d'oxygène dans le gaz d'échappement ou rend déterminable le taux de non-équilibre de monoxyde de carbone à l'aide d'un taux d'oxygène faible à négligeable.

3. Procédé selon la revendication 1 ou 2, **caractérisé en ce que**, pour la détermination du premier taux de monoxyde de carbone dans le gaz d'échappement caractérisant un taux d'équilibre

- un taux d'oxygène dans le gaz d'échappement est déterminé au moyen d'une règle de calcul et, à l'aide d'un taux d'oxygène dans le gaz d'échappement non négligeable et
- à l'aide de la valeur d'équilibre mentionnée, au moyen de la deuxième règle de calcul, un taux de monoxyde de carbone dans le gaz d'échappement est déterminé, qui caractérise un taux de monoxyde de carbone caractérisant un taux d'équilibre.

4. Procédé selon l'une des revendications 1 à 3, **caractérisé en ce que**, pour la tension de cellule, la fenêtre de temps d'intégrale est prédéterminée comme une fenêtre de temps d'intégrale déterminée comprenant un premier nombre de valeurs de tension de cellule et/ou une fenêtre de temps différentielle déterminée comprenant un deuxième nombre de valeurs de tension de cellule, dans lequel la fenêtre de temps différentielle comprend moins de valeurs de tension de cellule que la fenêtre de temps d'intégrale.

5. Procédé selon la revendication 1 ou 4, **caractérisé en ce que** la valeur d'équilibre est déterminée comme une valeur moyenne de toutes les valeurs de tension de cellule dans la fenêtre de temps d'intégrale et/ou la valeur de non-équilibre augmentée est déterminée comme la valeur moyenne ou la valeur d'enveloppe de toutes les valeurs de tension de cellule au-dessus de la zone de seuil et dans la fenêtre de temps différentielle.

6. Procédé selon l'une des revendications 1 à 5, **caractérisé en ce que** l'analyse de signaux isole des valeurs de tension de cellule de la sonde lambda les pointes de tension de cellule des valeurs de tension de cellule.

7. Procédé selon l'une des revendications 1 à 6, **caractérisé en ce que**, à partir de la tension de cellule

   - la valeur d'équilibre en dessous de la zone de seuil est déterminée en fonction du temps.

8. Procédé selon l'une des revendications 1 à 7, **caractérisé en ce que** seules les valeurs de tension de cellule qui dépassent la valeur d'équilibre d'un facteur supérieur ou égal à 1,5 sont utilisées pour la détermination de la valeur de non-équilibre augmentée.

9. Procédé selon la revendication 8, **caractérisé en ce que** la zone de seuil comprend un intervalle de valeurs de tension de cellule qui se trouvent entre 1 fois la valeur d'équilibre et 1,5 fois la valeur d'équilibre.

10. Procédé selon l'une des revendications 1 à 9, **caractérisé en ce que** seules les valeurs de tension de cellule qui présentent une largeur à mi-hauteur inférieure à 10 secondes et une fenêtre de temps d'intégrale qui présente une largeur, de préférence réglable, entre 2 secondes et 100 secondes, sont utilisées pour la détermination de la valeur de non-équilibre augmentée.

11. Procédé selon l'une des revendications 1 à 10, **caractérisé en ce que**, les valeurs de tension de cellule à utiliser pour la détermination de la valeur de non-équilibre augmentée apparaissent avec un taux moyen d'une à cinq valeurs par minute, dans lequel la fenêtre de temps d'intégrale est choisie de façon à ce qu'en moyenne, au moins deux valeurs de tension de cellule à utiliser pour la détermination de la valeur de non-équilibre augmentée apparaissent dans la fenêtre de temps d'intégrale, dans lequel la fenêtre de temps d'intégrale se trouve dans un intervalle au-dessus de 100 secondes.

12. Procédé selon l'une des revendications 1 à 11, **caractérisé en ce que** le premier taux de monoxyde de carbone dans le gaz d'échappement est déterminé à partir de la valeur d'équilibre et le deuxième taux de monoxyde de carbone dans le gaz d'échappement est déterminé à partir de la valeur de non-équilibre augmentée, au moyen de la même au moins une deuxième règle de calcul, dans lequel une application d'une équation de Nernst et une application d'une équation eau-gaz, pour une détermination du premier taux de monoxyde de carbone dans le gaz d'échappement à partir de la valeur d'équilibre, en option en tenant compte d'un taux d'oxygène non négligeable, a lieu de la même façon que pour une détermination du deuxième taux de monoxyde de carbone, en option sans tenir compte d'un taux d'oxygène négligeable.

13. Procédé selon l'une des revendications 1 à 12, **caractérisé en ce que** la valeur de non-équilibre augmentée est utilisée pour la détermination du deuxième taux de monoxyde de carbone dans le gaz d'échappement, caractérisant un taux de non-équilibre, n'est utilisée pour un procédé de commande et/ou de régulation d'un four industriel régénératif que lorsque la valeur de non-équilibre augmentée dépasse une zone de seuil.

14. Procédé selon l'une des revendications 1 à 13, **caractérisé en ce que** la valeur de non-équilibre augmentée est utilisée pour la détermination du deuxième taux de monoxyde de carbone dans le gaz d'échappement, caractérisant un taux de non-équilibre, n'est utilisée pour un procédé de commande et/ou de régulation d'un four industriel régénératif que lorsqu'une température de gaz d'échappement se trouve à au moins 10 K au-dessus d'une température normale.

15. Procédé selon l'une des revendications 1 à 14, **caractérisé en ce que**, pour l'étalonnage d'une mesure de monoxyde de carbone par rapport à une autre mesure de concentration en monoxyde de carbone indépendante au même endroit de mesure, un réglage du nombre réglable de valeurs de tension de cellule est utilisé de manière variable pour la fenêtre de temps d'intégrale pour l'enregistrement glissant dans l'analyse de signaux, plus particulièrement une unité d'analyse électronique.

16. Procédé selon l'une des revendications 1 à 15, **caractérisé en ce qu'**une hypothèse d'une différence de température entre une température de gaz d'échappement du gaz d'échappement analysé et la température de la sonde lambda elle-même est utilisée de manière variable, à savoir d'une part la température de la sonde lambda pour une utilisation dans la première règle de calcul, qui utilise la tension de cellule et la température de la sonde lambda sur la base d'une équation de Nernst et d'autre part la température du gaz d'échappement pour une utilisation dans la deuxième règle de calcul, qui utilise la composition du carburant sur la base d'une équation eau-gaz.

17. Dispositif de commande (1000) pour un four industriel régénératif (100), plus particulièrement pour un four de fusion de verre, conçu pour l'exécution du procédé selon l'une des revendications 1 à 16.

18. Four industriel régénératif (100), plus particulièrement four de fusion de verre, avec le dispositif de commande selon la revendication 17.

EP 2 679 991 B1

FIG. 1

FIG. 2

FIG. 3

FIG. 4A

FIG. 4B

FIG. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010041155 A1
- JP 2012026718 A
- DE 4323879 A1
- DE 3632480 A1